# FASCICULE DE BREVET EUROPEEN

(11) **EP 1 720 544 B1**
(45) Date de publication et mention de la délivrance du brevet: **19.03.2008**
(21) Numéro de dépôt: 05717646.3
(22) Date de dépôt: 18.02.2005
(51) Int. Cl.: A61K 31/403, C07D 209/52, C07D 403/12, A61P 25/00

(54) **NOUVEAUX DERIVES AZABICYCLIQUES, LEUR PROCEDE DE PREPARATION ET LES COMPOSITIONS PHARMACEUTIQUES QUI LES CONTIENNENT**
NEUE AZABICYCLISCHE DERIVATE, HERSTELLUNGSVERFAHREN DAFÜR UND SIE ENTHALTENDE PHARMAZEUTISCHE ZUSAMMENSETZUNGEN
NOVEL AZABICYCLIC DERIVATIVES, PREPARATION METHOD THEREOF AND PHARMACEUTICAL COMPOSITIONS CONTAINING SAME

(30) Priorité: 20.02.2004 FR 0401690
(43) Date de publication de la demande: 15.11.2006
(73) Titulaire: Les Laboratoires Servier, 92415 Courbevoie Cedex (FR)
(72) Inventeur: CASARA, Patrick, F-78670 Vilennes sur Seine (FR); CHOLLET, Anne-Marie, F-78230 Le Pecq (FR); DHAINAUT, Alain, F-78400 Chatou (FR); BERT, Lionel, F-78 160 Marly le Roi (FR); LESTAGE, Pierre, F-78170 La Celle Saint Cloud (FR); LOCKHART, Brian, F-78810 Feucherolles (FR)
(86) Numéro de dépôt international: PCT/FR2005/000382
(87) Numéro de publication internationale: WO 2005/089747

(56) Documents cités:
- WO-A-02/06223
- US-A- 5 703 091
- FAGHIH ET AL.: "Aminoalkoxybiphenylnitriles as Histamine-3 Receptor Ligands" BIOORGANIC AND MEDICINAL CHEMISTRY LETTERS, vol. 12, 2002, pages 3077-3079, XP002335013

## Description

La présente invention concerne de nouveaux dérivés azabicycliques, leur procédé de préparation et les compositions pharmaceutiques qui les contiennent.

Les composés de la présente invention sont particulièrement intéressants d'un point de vue pharmacologique pour leur interaction avec les systèmes histaminergiques centraux in vivo, trouvant leur application dans le traitement des neuropathologies associées au vieillissement cérébral, des troubles de l'humeur, du comportement alimentaire et du rythme veille-sommeil, ainsi que du syndrome d'hyperactivité avec déficits attentionnels.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, de récentes études neuropharmacologiques ont montré que l'histamine via les systèmes histaminergiques centraux jouait un rôle de neurotransmetteur ou neuromodulateur en situations physiologiques ou physiopathologiques (Pell et Green, Annu. Rev. Neurosci., 1986, 9 209-254; Schwartz et al., Physiol. Rev., 1991, 71, 1-51). Ainsi, il a été montré que l'histamine intervenait dans divers processus physiologiques et comportementaux tels que la thermorégulation, la régulation neuro-endocrinienne, le rythme circadien, les états cataleptiques, la motricité, l'agressivité, le comportement alimentaire, l'apprentissage et la mémorisation, ainsi que la plasticité synaptique (Hass et al., Histaminergic neurones: morphology and function, Boca Raton, FL : CRC Press, 1991, pp. 196-208 ; Brown et al., Prog. Neurobiology, 2001, 63, 637-672).

Des études, réalisées chez l'animal, ont montré que l'augmentation des taux endogènes extra-synaptiques d'histamine permettait de promouvoir les états de vigilance, les processus d'apprentissage et de mémoire, de réguler la prise alimentaire, et de s'opposer à des crises convulsives (Brown et al., Prog. Neurobiol., 2000, 63, 637-672; Passani et al., Neurosci. Biobehav. Rev., 2000, 24 107-113). En conséquence, les indications thérapeutiques potentielles pour des composés capables d'augmenter le turn-over ou la libération d'histamine au niveau central sont le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels. Par ailleurs, des travaux ont montré qu'une injection d'histamine au niveau des noyaux centraux hypothalamiques impliqués dans la régulation de la satiété atténue l'alimentation chez le rat. De plus, un hypofonctionnement de la transmission histaminergique a été mis en évidence chez des rats génétiquement obèses (Machidori et al., Brain Research, 1992, 590, 180-186). En conséquence, les troubles du comportement alimentaire et l'obésité sont également des indications thérapeutiques potentielles pour les composés de la présente invention.

Quelques documents décrivent des composés comportant un motif octahydrocyclopenta-[b]pyrrole ou octahydrocyclopenta[c]pyrrole [US 2,962,496 ; J. Chem. Soc., Chem. Commun., 1995, 10, 1009-1010; Tetrahedron, 1991, 47(28), 5161-5172 ; Tetrahedron Lett., 1988, 29 (28), 3481-3482 ; J. Med. Chem., 1973, 16(4), 394-397]. Certains de ces composés sont connus pour leur utilité dans le traitement de maladies cardiovasculaires notamment de l'hypertension ou comme anesthésiant local, d'autres ont été étudiés du point de vue mécanistique sur des réactions chimiques de type cycloaddition ou cyclisation intramoléculaire catalysée. En revanche, aucun document ne décrit ou ne suggère pour ces composés une activité in vivo en tant qu'activateurs des systèmes histaminergiques centraux, propriété originale des composés revendiqués par la Demanderesse.

Des ligands des récepteurs histaminergiques centraux de type H3 sont décrits dans la demande WO 02/06223 ainsi que dans Bioorganic 5 Medicinal Chemistry Letters, 12, (2002), 3077-3079.

Plus particulièrement, la présente invention concerne les composés de formule (I) : 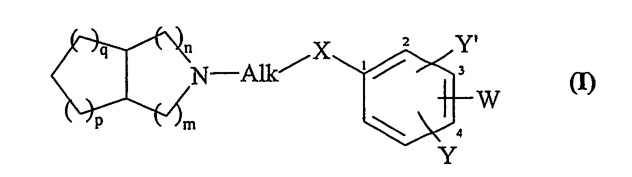 dans laquelle :
- **m** et **n,** identiques ou différents, représentent un entier compris inclusivement entre 0 et 2 avec la somme des deux entiers comprise inclusivement entre 2 et 3,
- **p** et **q**, identiques ou différents, représentent un entier compris inclusivement entre 0 et 2,
- **Alk** représente une chaîne alkylène, alkénylène ou alkynylène,
- **Y** et **Y**', identiques ou différents, représentent un atome d'hydrogène, d'halogène, ou un groupement alkyle, alkoxy, alkylthio, alkylsulfinyle, alkylsulfonyle, mercapto, hydroxy, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo ou cyano,
- **X** représente un atome d'oxygène, de soufre ou un groupement -N(R)-, avec R représentant un atome d'hydrogène ou un groupement alkyle,
- **W** représente un groupement choisi parmi cyano (lorsque X représente un atome d'oxygène ou un groupement -N(R)-), N(R₁)-Z₁-R₂ et -Z₂-NR₁R₂,
   avec :
   - Z₁ représentant -C(O)-, -C(S)-, -C(NR₄)-, *-C(O)-N(R₃)-, *-C(S)-N(R₃)-, *-C(NR₄)-N(R₃)-, *-C(O)-O-, *-C(S)-O-, -S(O)ᵣ-, avec r = 1 ou 2, et * correspondant au point d'attachement à N(R₁),
   - Z₂ représentant -C(O)-, -C(S)-, -C(NR₄), -S(O)ᵣ-, liaison,
   - R₁ R₂, R₃ et R₄, identiques ou différents, représentant un atome d'hydrogène, un groupement alkyle éventuellement substitué, alkényle éventuellement substitué, alkynyle éventuellement substitué, alkoxy, cycloalkyle éventuellement substitué, un groupement hétérocycloalkyle éventuellement substitué, un groupement aryle éventuellement substitué ou hétéroaryle éventuellement substitué,
   - ou alors R₁ et R₂ ou R₂ et R₃ forment ensemble avec le ou les atomes qui les portent, un groupement hétérocycloalkyle éventuellement substitué, ou hétéroaryle éventuellement substitué,
   leurs énantiomères, diastéréoisomères, ainsi que leurs sels d'addition à un ou plusieurs acides ou à une ou plusieurs bases pharmaceutiquement acceptables,
   étant entendu que :
   - le terme alkyle désigne une chaîne hydrocarbonée, linéaire ou ramifiée, contenant de 1 à 6 atomes de carbone,
   - le terme alkényle désigne un groupement linéaire ou ramifié contenant de 3 à 6 atomes de carbone et de 1 à 3 doubles liaisons,
   - le terme alkynyle désigne un groupement linéaire ou ramifié contenant de 3 à 6 atomes de carbone et de 1 à 3 triples liaisons,
   - le terme alkoxy désigne un groupement alkyle-oxy dont la chaîne alkyle, linéaire ou ramifiée, contient de 1 à 6 atomes de carbone,
   - le terme aryloxy éventuellement substitué désigne un groupement aryle-oxy dont le groupement aryle est éventuellement substitué,
   - le terme acyle désigne un groupement RₐC(O)- où Rₐ représente un atome d'hydrogène ou un groupement alkyle,
   - le terme perhalogénoalkyle désigne une chaîne carbonée, linéaire ou ramifiée, contenant de 1 à 3 atomes de carbone et de 1 à 7 atomes d'halogène,
   - le terme alkylène désigne un radical bivalent, linéaire ou ramifié, contenant de 1 à 6 atomes de carbone,
   - le terme alkénylène désigne un radical bivalent, linéaire ou ramifié, contenant de 2 à 6 atomes de carbone et de 1 à 3 doubles liaisons,
   - le terme alkynylène désigne un radical bivalent linéaire ou ramifié, contenant de 2 à 6 atomes de carbones et de 1 à 3 triples liaisons,
   - le terme aryle désigne un groupement phényle, naphtyle, indanyle, indényle, dihydronaphtyle ou tétrahydronaphtyle,
   - le terme hétéroaryle désigne un groupement monocyclique ou bicyclique dans lequel au moins un des cycles est aromatique, comportant de 5 à 11 chaînons et de 1 à 4 hétéroatomes, choisis parmi l'azote, l'oxygène et le soufre,
   - le terme cycloalkyle désigne un monocycle ou bicycle hydrocarboné comportant de 3 à 11 atomes de carbone, et éventuellement insaturé par 1 ou 2 insaturations,
   - le terme hétérocycloalkyle désigne un groupement mono ou bicyclique, saturé ou insaturé par 1 ou 2 insaturations, contenant de 4 à 11 chaînons et possédant de 1 à 4 hétéroatomes choisis parmi azote, oxygène et soufre,
   - l'expression "éventuellement substitué" affectée aux termes cycloalkyle, aryle, hétéroaryle, hétérocycloalkyle, signifie soit i) que ces groupements peuvent être substitués par 1 à 3 substituants, identiques ou différents, choisis parmi alkyle, alkoxy, alkylthio, alkylsulfinyle, alkylsulfonyle, halogène, hydroxy, mercapto, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo et cyano, soit ii) que ces groupements peuvent être substitués par un groupement aryle, hétéroaryle, cycloalkyle, héterocycloalkyle ou benzyle ; étant entendu que les groupements aryle ou hétéroaryle peuvent être en plus substitués par un ou deux groupements oxo sur la partie non aromatique des groupes ayant une partie aromatique et une partie non aromatique, et les groupements cycloalkyle ou hétérocycloalkyle peuvent être substitués également par un ou deux groupements oxo,
   - l'expression « éventuellement substitué » affectée au terme alkyle, alkényle, ou alkynyle, signifie que ces groupements peuvent être substitués par un ou deux groupements, identiques ou différents, choisis parmi alkylthio, alkylsulfinyle, alkylsulfonyle, alkoxy, halogène, hydroxy, mercapto, nitro, amino, acyle, aminocarbonyle, acylamino, akoxycarbonyle, carboxy, sulfo, cyano, aryle éventuellement substitué, hétéroaryle éventuellement substitué, cycloalkyle éventuellement substitué, hétérocycloalkyle éventuellement substitué, et aryloxy éventuellement substitué.

Parmi les acides pharmaceutiquement acceptables, on peut citer à titre non limitatif, les acides chlorhydrique, bromhydrique, sulfurique, phosphonique, acétique, trifluoroacétique, lactique, pyruvique, malonique, oxalique, succinique, glutarique, fumarique, tartrique, maléique, citrique, ascorbique, méthane sulfonique, camphorique, etc...

Parmi les bases pharmaceutiquement acceptables, on peut citer à titre non limitatif, l'hydroxyde de sodium, l'hydroxyde de potassium, la triéthylamine, etc...

Les groupements préférés aryle sont le groupement phényle.

Avantageusement, les composés de l'invention sont ceux pour lesquels dans la formule (I) q est égal à 1.

Un aspect avantageux de l'invention concerne les composés pour lesquels n représente 1.

Des composés préférés de l'invention sont ceux pour lesquels m est égal à 1.

D'autres composés préférés de l'invention sont ceux pour lesquels m est égal à 2.

Des composés préférés de l'invention sont ceux pour lesquels p est égal à 1.

D'autres composés préférés de l'invention sont ceux pour lesquels p est égal à 2.

Un aspect particulièrement avantageux de l'invention concerne les composés de formule (I) pour lesquels X représente un atome d'oxygène ou de soufre (plus avantageusement d'oxygène).

Un autre aspect particulièrement avantageux de l'invention concerne les composés de formule (I) pour lesquels X représente un groupement -N(R)- (plus avantageusement NH).

Un aspect préféré est celui pour lequel les composés de l'invention de formule (I) comportent un groupement Y et Y' représentent chacun un atome d'hydrogène.

Un autre aspect préféré de l'invention est celui pour lequel les composés de l'invention de formule (I) comportent un groupement Y représentant un atome d'hydrogène et Y' représentant un atome d'halogène ou un groupement alkyle, alkoxy, alkylthio, alkylsulfinyle, alkylsulfonyle, mercapto, hydroxy, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo ou cyano. Plus préférentiellement Y' représentant un atome d'halogène.

Parmi les composés particulièrement avantageux se trouvent les composés de l'invention pour lesquels Alk représente une chaîne alkylène (plus particulièrement propylène).

Un aspect particulièrement avantageux de l'invention concerne les composés de formule (I) pour lesquels W est positionné sur le groupement phényle en position 4.

Un autre aspect avantageux de l'invention concerne les composés de formule (I) pour lesquels W représente un groupement cyano.

De manière avantageuse les composés de formule (I) sont ceux pour lesquels W représente un groupement -N(R₁)-Z₁-R₂.

D'une autre manière avantageuse les composés de formule (I) sont ceux comportant W qui représente un groupement -Z₂-NR₁R₂.

Les groupements préférés Z₂ sont choisis parmi -C(O)-, -C(S)-, -C(NR₄)- et -S(O)ᵣ-. Plus préférentiellement Z₂ représente un groupement -C(O)-.

D'autres composés préférés de l'invention sont ceux pour lesquels Z₂ représente une liaison.

Les groupements préférés Z₁ sont choisis parmi -C(O)-, -C(S)-, *-C(O)-N(R₃)-, *-C(S)-N(R₃)-, *-C(O)-O- et -S(O)₂-. Préférentiellement -C(O)- et *-C(O)-N(R₃)-(plus préférentiellement -C(O)-).

Un aspect particulièrement avantageux de l'invention concerne les composés de formule (I) pour lesquels R₁, R₂, R₃ et R₄, identiques ou différents, représentent :
- un atome d'hydrogène ;
- un groupement alkoxy ;
- un groupement cycloalkyle (préférentiellement cyclopropyle, cyclobutyle, ou cyclohexyle) ;
- un groupement phényle éventuellement substitué (préférentiellement par un ou deux substituants choisis parmi nitro, halogène, trihalogénoalkyle, alkyle et alkoxy) ;
- un groupement naphtyle ;
- un groupement hétéroaryle (préférentiellement choisi parmi thiènyle, furyle, pyridyle, benzofuryle et méthylènedioxyphényle) ;
- un groupement alkyle ;
- ou un groupement alkyle substitué :
   - soit par un groupement phényle éventuellement substitué (préférentiellement par un ou deux substituants choisis parmi halogène, trihalogénoalkyle, alkyle et alkoxy),
   - soit par un groupement cycloalkyle (préférentiellement cyclopropyle),
   - soit par un groupement hétérocycloalkyle (préférentiellement morpholinyle, piperazinyle, piperidinyle),
   - soit par un groupement hétéroaryle (préférentiellement thiènyle, furyle, pyridyle, imidazolyle),
   - soit par un ou deux groupements alkoxy (préférentiellement méthoxy), ou
   - soit par un groupement phényloxy.

Un autre aspect particulièrement avantageux de l'invention concerne les composés de formule (I) pour lesquels W représente un groupement choisi parmi N(R₁)-C(O)-NR₂R₃ ; N(R₁)-C(S)-NR₂R₃ ; -C(O)-NR₁R₂ et -C(S)-NR₁R₂ ; où R₁ et R₂ ou R₂ et R₃ forment ensemble avec le ou les atomes qui les portent un groupement hétérocycloalkyle ou un groupement pipéridinopipéridinyle.
Les groupements hétérocycloalkyle préférés sont soit monocycliques saturés à 6 ou 7 chaînons contenant éventuellement en plus de l'atome d'azote un autre hétéroatome choisi parmi l'azote, l'oxygène et le soufre ; soit bicycliques saturés à 6 à 10 chaînons contenant éventuellement en plus de l'atome d'azote un autre hétéroatome choisi parmi l'azote, l'oxygène et le soufre.

Un autre aspect particulièrement avantageux de l'invention concerne les composés de formule (I) pour lesquels W représente un groupement -Z₂-NR₁R₂ où Z₂ représente une liaison ; R₁, R₂ forment ensemble avec l'atome d'azote qui les porte un groupement hétéroaryle (préférentiellement imidazolyle ou triazolyle) ou alors R₁ représente un atome d'hydrogène ou un groupement alkyle et R₂ un groupement aryle ou hétéroaryle (préférentiellement hétéroaryle, plus préférentiellement un groupement choisi parmi quinazolyle, isoquinolyle, quinolyle, et purinyle).

De manière avantageuse les composés de formule (I) sont ceux pour lesquels W représente un groupement -C(O)-NR₁R₂ où R₁, R₂ forment ensemble avec l'atome d'azote qui les porte un groupement choisi parmi pipérazinyle éventuellement substitué par un groupement alkyle ou benzyle ; pipéridinyle éventuellement substitué par un groupement alkyle ou benzyle ; azépanyle ; morpholinyle ; thiomorpholinyle ; octahydrocyclopentapyrrolyle ; dihydroquinolinyle ; et tétrahydroquinolinyle.

Un aspect particulièrement avantageux de l'invention concerne les composés de formule (I) pour lesquels W représente un groupement -C(O)-NR₁R₂ où R₁, R₂ représentent indépendamment un groupement alkyle ou un atome d'hydrogène.

Un autre aspect particulièrement avantageux de l'invention concerne les composés de formule (I) pour lesquels W représente un groupement N(R₁)-C(O)-R₂ où R₁, R₂ représentent indépendamment un groupement alkyle ou un atome d'hydrogène.

Parmi les composés préférés de l'invention, on peut citer plus particulièrement le 4-(3-hexahydrocyclopenta[*c*]pyrrol-2(1*H*)ylpropoxy)benzonitrile, le 4-[(3-hexahydrocyclopenta[*c*]-pyrrol-2(1*H*)-ylpropoxy)benzamide, le 4-[3-(hexahydrocyclopenta[*c*]pyrrol-2(1*H*)-yl)propoxy]-*N*-méthylbenzamide, le 4-[3-(hexahydrocyclopenta[*c*]pyrrol-2(1*H*)-yl)propoxy]-*N*,*N* diméthylbenzamide et le *N*-[4-(3-hexahydrocyclopenta[*c*]pyrrol-2(1*H*)-ylpropoxy)phényl] acétamide.

L'invention concerne également le procédé de préparation des composés de formule (I), caractérisé en ce que l'on utilise comme produit de départ un composé de formule (II) : 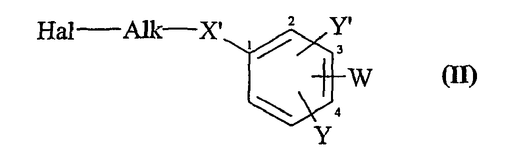 dans laquelle :
Alk est tel que défini dans la formule (I), Hal représente un atome d'halogène, X' représente un atome d'oxygène, de soufre ou un groupement -N(p)-, où (p) représente un atome d'hydrogène, un groupement de protection classique de l'atome d'azote ou un groupement alkyle, W, Y et Y' sont tels que définis dans la formule (I),
composé de formule (II) qui après déprotection éventuelle, se condense en milieu basique avec un bicycle de formule (III) : 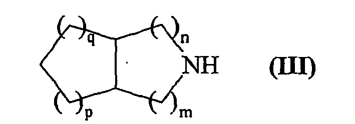 dans laquelle :
n, m, p et q sont tels que définis dans la formule (I), pour conduire au composé de formule (I),

■ composé de formule (I) qui, lorsque W représente un groupement cyano, réagit éventuellement avec la soude ou la potasse, pour conduire au composé de formule (I/b) : 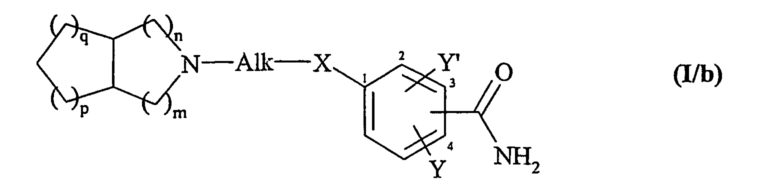 cas particulier des composés de formule (I) pour lequel Alk, n, m, p, q, X, Y et Y' sont tels que définis dans la formule (I),
composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un ou plusieurs acides ou à une ou plusieurs bases pharmaceutiquement acceptables,
étant entendu :
- qu'à tout moment jugé opportun au cours du procédé précédemment décrit, le ou les groupements carbonyle, thiocarbonyle, amino, alkylamino du réactif de départ (II) peuvent être protégés puis, après condensation, déprotégés pour les besoins de la synthèse,
- que les réactifs (II), et (III) sont préparés selon des modes opératoires connus, décrits dans la littérature.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par des techniques spectroscopiques et spectrométriques usuelles.

Les produits de départ utilisés sont des produits connus ou préparés selon des modes opératoires connus.

### PREPARATION 1 : N-(4-Chlorobutyl)-N-(4-cyanophényl)acétamide

9 g (54,1 mmol) de *N*-(4-cyanophényl)acétamide sont mis en solution dans 100 ml de THF. Le mélange est refroidi à 0°C avant l'addition goutte à goutte de 51 ml d'une solution 1,6 N dans l'hexane de nBuLi (1,5 éq). La solution est laissée remonter à température ambiante pendant 1 heure puis refroidie à 0°C avant l'ajout goutte à goutte de 9,9 ml de 1-chloro-4-iodobutane (81 mmol). La réaction est agitée à température ambiante pendant 18 h puis hydrolysée par une solution aqueuse saturée de chlorure d'ammonium (100 ml) et extraite par de l'acétate d'éthyle. Les phases organiques sont rassemblées, séchées sur sulfate de magnésium et concentrées. Une purification par chromatographie sur silice (éluant : éther de pétrole/acétate d'éthyle : 1/1) conduit à une huile jaune contenant le produit attendu.

### PREPARATION 2 : N-(3-Chloropropyl)-N-(4-cyanophényl)acétamide

Le procédé expérimental est identique à celui de la Préparation 1 en remplaçant le 1-chloro-4-iodobutane par le 1-chloro-3-iodopropane.

### PREPARATION 3 : N-(2-Chloroéthyl)-N-(4-cyanoghényl)acétamide

Le procédé expérimental est identique à celui de la Préparation 1 en remplaçant le 1-chloro-4-iodobutane par le 1-chloro-2-iodoéthane.

### EXEMPLE 1 : Oxalate du 4-(3-hexahydrocyclopenta[c]pyrrol-2(1H)-ylpropoxy)-benzonitrile

### Stade 1 : 4-(3-Chloropropoxy)benzonitrile

Un mélange de 0,47 g (0,004 mole) de 4-hydroxybenzonitrile, 0,63 g (0,004 mole) de 1-bromo-3-chloropropane et 1,95 g (0,006 mole) de carbonate de césium dans 10 ml d'acétonitrile est chauffé au reflux pendant 5 heures.

### Stade 2 : Oxalate du 4-(3-hexahydrocyclopenta[c]pyrrol-2(1H)-ylpropoxy)-benzonitrile

Dans le milieu réactionnel du stade 1 à température ambiante sont ajoutés 0,44 g (0,004 mole) d'octahydrocyclopenta[c]pyrrole* et 0,30 g (0,002 mole) d'iodure de sodium et le chauffage au reflux est repris pendant 16 heures. Le précipité est filtré, rincé avec de l'acétonitrile. Le filtrat est concentré à sec. Le résidu est repris dans du dichlorométhane. Cette solution est extraite avec de la soude, puis de l'eau, séchée sur sulfate de magnésium et concentrée à sec. Le résidu est purifié par technique de chromatographie préparative sur phase Lichroprep RP-18. Le produit du titre est recristallisé dans l'éthanol sous forme d'oxalate.
*L'octahydrocyclopenta[c]pyrrole a été synthétisée selon la méthode de Roussi et Zang (Tetrahedron Lett., 1988, 29, 3481).
*ESI*⁺ *: [M*+*H]*⁺ *271,1810 (théorie: 271,1810)*

### EXEMPLE 2 : Oxalate du 4-(2-hexahydrocyclopenta[c]pyrrol-2(1H)-yléthoxy)-benzonitrile

Le procédé expérimental est identique à celui de l'Exemple 1 en remplaçant au stade 1 le 1-bromo-3-chloropropane, par le 1-bromo-2-chloroéthane.

| *Microanalyses élémentaires :* | | | |
|---|---|---|---|
| | C% | *H%* | *N%* |
| *Calculé :* | *62,42* | *6,40* | *8, 09* |
| *Trouvé :* | *62, 09* | *6, 38* | *8, 09* |

### EXEMPLE 3 : Oxalate du 4-(4-hexabydrocyclopenta[c]pyrrol-2(1H)-ylbutoxy)-benzonitrile

Le procédé expérimental est identique à celui de l'Exemple 1 en remplaçant au stade 1 le 1-bromo-3-chloropropane, par le 1-bromo-4-chlorobutane.

| *Microanalyses élémentaires :* | | | |
|---|---|---|---|
| | *C%* | *H%* | *N%* |
| *Calculé:* | *63, 28* | *6, 89* | *7, 31* |
| *Trouvé:* | *63,14* | *6, 78* | *6, 91* |

### EXEMPLE 4 : Oxalate du N-[4-(3-hexahydrocyclopenta[c]pyrrol-2(1H)-ylpropoxy)-phényl]acétamide

Le procédé expérimental est identique à celui de l'Exemple 1 en remplaçant au stade 1 le 4-hydroxybenzonitrile, par le *N*-(4-hydroxyphényl)acétamide.

| | |
|---|---|
| RMN¹H (DMSO D₆) : δ (ppm) : | 1,40-1,80 *(m,*6*H)* ; 2,00 *(s,*3*H)* ; 2,10 (quint,2*H*); 2,80 *(m,*4*H)* ; 3,25 *(t,*2*H)* ; 3,60 *(m,*2*H)* ; 4,00 (t*,*2*H*) ; 6,90 (d,2*H*) ; 7,50 (d,2*H*) ; 9,80 (s,1*H*). |

### EXEMPLE 5 : Oxalate du N-[3-(3-hexahydrocyclopenta[c]pyrrol-2(1H)-ylpropoxy)-phényl]acétamide

Le procédé expérimental est identique à celui de l'Exemple 1 en remplaçant au stade 1 le 4-hydroxybenzonitrile, par le *N*-(3-hydroxyphényl)acétamide.

| *Microanalyses élémentaires :* | | | |
|---|---|---|---|
| | *C%* | *H%* | *N%* |
| *Calculé :* | *61,21* | *7,19* | *7,14* |
| *Trouvé :* | *61, 06* | *7,28* | *7, 06* |

### EXEMPLE 6 : N-Ethyl-4-(3-hexahydracyclopenta[c]pyrrol-2(1H)-ylpropoxy)-benzamide

Le procédé expérimental est identique à celui de l'Exemple 1 en remplaçant au stade 1 le 4-hydroxybenzonitrile, par le *N*-éthyl-4-hydroxybenzamide.

| *Microanalyses élémentaires :* | | | |
|---|---|---|---|
| | *C%* | *H%* | *N%* |
| *Calculé :* | *72,12* | *8,92* | *8,85* |
| *Trouvé :* | *72,52* | *9,10* | *8,80* |

### EXEMPLE 7 : Oxalate du N-cyclopentyl-4-(3-hexahydrocyclopenta[c]pyrrol-2(1H)-ylprapoxy)benzamide

Le procédé expérimental est identique à celui de l'Exemple 1 en remplaçant au stade 1 le 4-hydroxybenzonitrile, par le *N*-cyclopentyl-4-hydroxybenzamide.

### EXEMPLE 8 : Oxalate du N-cyclopentyl-N-ethyl-4-(3-hexabtydrocyclopenta[c]-pyrrol-2(1H)-ylpropoxy)benzamide

Le procédé expérimental est identique à celui de l'Exemple 1 en remplaçant au stade 1 le 4-hydroxybenzonitrile, par le *N*-cyclopentyl-*N*-éthyl-4-hydroxybenzamide.

### EXEMPLE 9 : Oxalate du N,N-diéthyl-4-(3-hexahydrocyclopenta[c]pyrrol-2(1H)-yl-propoxy)benzamide

Le procédé expérimental est identique à celui de l'Exemple 1 en remplaçant au stade 1 le 4-hydroxybenzonitrile, par le *N,N*-diéthyl-4-hydroxybenzamide.

| *Microanalyses élémentaires :* | | | |
|---|---|---|---|
| | *C%* | *H%* | *N%* |
| *Calculé:* | *63,57* | *7,89* | *6,45* |
| *Trouvé :* | *63,37* | *7,93* | *6,34* |

### EXEMPLE 10 : Oxalate du N,N-dicyclopropyl-4-(3-hexahydrocyclopenta[c]pyrrol-2(1H)-ylpropoxy)benzamide

Le procédé expérimental est identique à celui de l'Exemple 1 en remplaçant au stade 1 le 4-hydroxybenzonitrile, par le *N,N*-dicyclopropyl-4-hydroxybenzamide.

### EXEMPLE 11 : Oxalate du 2-{3-[4-(1-azépanylcarbonyl)phénoxy]propyl}octahydrocyclopenta[c]pyrrole

Le procédé expérimental est identique à celui de l'Exemple 1 en remplaçant au stade 1 le 4-hydroxybenzonitrile, par le 4-(1-azépanylcarbonyl)phénol.

### EXEMPLE 12 : Oxalate du 2-{3-[4-(thiomorpholinocarbonyl)phénoxy]propyl}-octahydrocyclopenta[c]pyrrole

Le procédé expérimental est identique à celui de l'Exemple 1 en remplaçant au stade 1 le 4-hydroxybenzonitrile, par le 4-(thiomorpholinocarbonyl)phénol.

### EXEMPLE 13 : Oxalate du 2-(3-[4-(morpholinocarbonyl)phénoxy]propyl}-octahydrocyclopenta[c] pyrrole

Le procédé expérimental est identique à celui de l'Exemple 1 en remplaçant au stade 1 le 4-hydroxybenzonitrile, par le 4-(morpholinocarbonyl)phénol.

| *Microanalyses élémentaires :* | | | |
|---|---|---|---|
| | *C%* | *H%* | *N%* |
| *Calculé :* | *61,59* | *7,19* | *6,25* |
| *Trouvé :* | *61,50* | *7,21* | *6,30* |

### EXEMPLE 14 : Oxalate du 2-{3-[4-(1-pipérazinylcarbonyl)phénoxy]propyl}-octahydrocyclopenta[c] pyrrole

Le procédé expérimental est identique à celui de l'Exemple 1 en remplaçant au stade 1 le 4-hydroxybenzonitrile, par le 4-(1-pipérazinylcarbonyl)phénol.

### EXEMPLE 15 : Oxalate de la 2-[4-(3-hexahydrocyclopenta[c]pyrrol-2(1H)-yl propoxy)benzoyl]isoindoline

Le procédé expérimental est identique à celui de l'Exemple 1 en remplaçant au stade 1 le 4-hydroxybenzonitrile, par le 4-(1,3-dihydro-2H isoindol-2-ylcarbonyl)phénol.

### EXEMPLE 16 : Oxalate de la 5-bromo-2-[4-(3-hexahydrocyclopenta[c]pyrrol-2(1H)-ylpropoxy)benzoyl]isoindoline

Le procédé expérimental est identique à celui de l'Exemple 1 en remplaçant au stade 1 le 4-hydroxybenzonitrile, par le 4-[(5-bromo-1,3-dihydro-2*H*-isoindol-2-yl)carbonyl]phénol.

### EXEMPLE 17 : Oxalate du 2-{3-[4-(hexahydrocyclopenta[c]pyrrol 2(1H)-ylcarbonyl)phenoxy]propyl}octahydrocyclopenta[c]pyrrole

Le procédé expérimental est identique à celui de l'Exemple 1 en remplaçant au stade 1 le 4-hydroxybenzonitrile, par le 4-(hexahydrocyclopenta[*c*]pyrrol-2(1*H*)-ylcarbonyl)phénol.

| *Microanalyses élémentaires :* | | | |
|---|---|---|---|
| | *C%* | *H%* | *N%* |
| *Calculé :* | *62,65* | *7,21* | *5,41* |
| *Trouvé :* | *63,14* | *7,30* | *5,47* |

### EXEMPLE 18 : Oxalate du 4-[(4-hexahydrocyclopenta[c]pyrrol-2(1H)-ylbutyl)-amino]benzonitrile

### Stade 1 : N-(4-Cyanophényl)-N-(4-hexahydrocyclopenta[c]pyrrol-2(1H)-ylbutyl)-acétamide

2 g (8 mmol) du dérivé chloré synthétisé dans la Préparation 1 sont mis en solution dans 65 ml d'éthanol avec 1,5 g d'octahydrocyclopenta[c]pyrrole (2 éq) et 12 mg de NaI (0,01 éq). Le mélange est chauffé 18 heures à reflux avant d'être évaporé à sec sous vide. Le résidu est repris par de l'acétate d'éthyle puis lavé par de la soude normale. La phase organique est séchée sur sulfate de magnésium, concentrée et purifiée par colonne chromatographique sur silice (éluant : dichlorométhane-éthanol : 9/1) pour fournir 1,4 g du produit attendu.

### Stade 2: Oxalate du 4-[(4-hexahydrocyclopenta[c]pyrrol-2(1H)-ylbutyl)amino]benzonitrile

A une solution du composé préparé au stade précédent (423 mg) dans 2,6 ml d'éthanol sont ajoutés 133 mg (1,5 éq) d'éthanolate de sodium. Le mélange est porté à reflux pendant 5 heures puis concentré sous vide. Le résidu est repris par du dichlorométhane, lavé par de l'eau, puis séché sur sulfate de magnésium avant évaporation du solvant. Une purification par colonne chromatographique (éluant : dichlorométhane/éthanol/ammoniaque : 10/0,5/0,25) permet d'obtenir 330 mg de produit. 260 mg de ce composé sont mis en solution dans de l'éthanol puis l'ajout de 2,5 équivalent d'acide oxalique en solution dans l'éthanol conduit à la précipitation du sel.
*ESI*⁺ *: [M*+*H]*⁺ *284,2085 (théorie : 284,2127)*

### EXEMPLE 19 : Oxalate du 4-[(3-hexahydrocyclopenta[c]pyrrol-2(1H)-ylpropyl)-amino]benzonitrile

Le procédé expérimental est identique à celui de l'Exemple 18 en remplaçant le réactif de la Préparation 1 par celui de la Préparation 2.

| *Microanalyses élémentaires :* | | | |
|---|---|---|---|
| | *C%* | *H%* | *N%* |
| *Calculé :* | *63,49* | *7,01* | *11,69* |
| *Trouvé :* | *63, 22* | *7, 04* | *11,47* |

### EXEMPLE 20 : Oxalate du 4-[(2-hexahydrocyclopenta[c]pyrrol-2(1H-yléthyl)-amino]benzonitrile

Le procédé expérimental est identique à celui de l'Exemple 18 en remplaçant le réactif de la Préparation 1 par celui de la Préparation 3.

| *Microanalyses élémentaires :* | | | |
|---|---|---|---|
| | *C%* | *H%* | *N,%* |
| *Calculé :* | *60,81* | *6,49* | *11,56* |
| *Trouvé :* | *60,60* | *6,00* | *11,30* |

### EXEMPLE 21 : Oxalate du 4-[(4-hexahydrocyclopenta[c]pyrrol-2(1H)-ylbutyl)-amino]benzamide

436 mg du composé de l'exemple 18 sont mis en solution dans 4 ml d'éthanol. 86 mg de potasse (1 éq) sont dissous dans 1,5 ml d'eau avant d'être ajoutés à la solution alcoolique. Le milieu est porté à reflux pendant 1,5 heure puis évaporé à sec. Le résidu est repris par du dichlorométhane. Cette solution est lavée à l'eau, séchée sur sulfate de magnésium puis concentrée sous vide. Le produit est cristallisé sous forme d'oxalate.
*ESI*⁺*: [M*+*H]*⁺ *302,2212 (théorie : 302,2232)*

### EXEMPLE 22 : Oxalate du 4-(3-herahydrocyclopenta[c]gyrrol-2(1H)-ylpropoxy)-benzamide

Le procédé expérimental est identique à celui de l'Exemple 21 en partant du composé de l'Exemple 1.

| *Microanalyses élémentaires :* | | | |
|---|---|---|---|
| | *C%* | *H%* | *N%* |
| *Calculé :* | *60,30* | *6,93* | *7,40* |
| *Trouvé :* | *60,21* | *6,65* | *7,31* |

### EXEMPLE 23 : Oxalate du 4-[(3-hexahydrocyclopenta[c]pyrrol-2(1H)-ylpropyl)-amino]benzamide

Le procédé expérimental est identique à celui de l'Exemple 21 en partant du composé de l'Exemple 19.

### EXEMPLE 24 : Oxalate du 4-[(2-hexahydrocyctopenta[c]pyrrol-2(1H)-yléthyl)-amino]benzamide

Le procédé expérimental est identique à celui de l'Exemple 21 en partant du composé de l'Exemple 20.

### EXEMPLE 25 : Oxalate du N-{4-[3-(hexahydrocyclopenta[c]pyrrol-2(1H)-yl)propoxy]phényl}-2-méthylpropanamide

Le procédé expérimental est identique à celui de l'Exemple 31 en remplaçant au stade 2 le chlorure de 4-fluorobenzoyle, par le chlorure de 2-méthylpropanoyle.

| *Microanalyses élémentaires :* | | | |
|---|---|---|---|
| | *C%* | *H%* | *N%* |
| *Calculé:* | *61,20* | *7,66* | *6,45* |
| *Trouvé :* | *61,32* | *7,47* | *6,24* |

### EXEMPLE 26 :N-{4-[3-(Hexahydrocyctopenta[c]pyrrol-2(1H)-yl)propoxylphényl}-2,2-diméthylpropanamide

Le procédé expérimental est identique à celui de l'Exemple 31 en remplaçant au stade 2 le chlorure de 4-fluorobenzoyle, par le chlorure de 2,2-diméthylpropanoyle.

| *Microanalyses élémentaires :* | | | |
|---|---|---|---|
| | *C%* | *H%* | *N%* |
| *Calculé :* | *73,22* | *9,36* | *8,13* |
| *Trouvé :* | *73,69* | *9,33* | *8,20* |

### EXEMPLE 27 : N-{4-[3-(Hexahydrocyclopenta[c]pyrrol-2(1H)-yl)propoxy]-phényl}cyclopropanecarboxamide

Le procédé expérimental est identique à celui de l'Exemple 31 en remplaçant au stade 2 le chlorure de 4-fluorobenzoyle, par le chlorure de cyclopropanecarbonyle.

| *Microanalyses élémentaires :* | | | |
|---|---|---|---|
| | *C%* | *H%* | *N%* |
| *Calculé :* | *73,14* | *8,59* | *8,53* |
| *Trouvé:* | *72,04* | *8,67* | *8,31* |

### EXEMPLE 28: N-{4-[3-(Hexahydrocyclopenta[c]pyrrol-2(1H)propoxy]-phényl}cyclobutanecarboxamide

Le procédé expérimental est identique à celui de l'Exemple 31 en remplaçant au stade 2 le chlorure de 4-fluorobenzoyle, par le chlorure de cyclobutanecarbonyle.

| *Microanalyses élémentaires :* | | | |
|---|---|---|---|
| | *C%* | *H%* | *N%* |
| *Calculé :* | *73, 65* | *8,83* | *8,18* |
| *Trouvé :* | *73, 24* | *8,68* | *8,12* |

### EXEMPLE 29: N-{4-[3-(Hexabydrocyclopenta[c]pyrrol-2(1H)-yl)propoxy]-phényl}cyclohexanecarboxamide

Le procédé expérimental est identique à celui de l'Exemple 31 en remplaçant au stade 2 le chlorure de 4-fluorobenzoyle, par le chlorure de cyclohexanecarbonyle.

| *Microanalyses élémentaires :* | | | |
|---|---|---|---|
| | *C%* | *H%* | *N%* |
| *Calculé :* | *74,56* | *9,25* | *7,56* |
| *Trouvé :* | *74,20* | *9,38* | *7,40* |

### EXEMPLE 30 : N-{4-[3-(Hexahydrocyclopenta[c]pyrrol-2(1H)-yl)propoxy]phényl}-4-nitrobenzamide

Le procédé expérimental est identique à celui de l'Exemple 31 en remplaçant au stade 2 le chlorure de 4-fluorobenzoyle, par le chlorure de 4-nitrobenzoyle.

| *Microanalyses élémentaires :* | | | |
|---|---|---|---|
| | *C%* | *H%* | *N%* |
| *Calculé :* | *67,46* | *6,65* | *10,26* |
| *Trouvé :* | *68,18* | *6,60* | *10,31* |

### EXEMPLE 31 : N-{4-[3-(-Hexahydrocyclopenta[c]pyrrol-2(1H)-yl)propoxy]phényl}-4-fluorobenzamide

### Stade 1 : 4-[3-(hexahydrocyclopenta[c]pyrrol-2(1H)-yl)propoxy]aniline

Le composé du titre est obtenu par hydrolyse acide de 1,5 g du composé de l'exemple 4 en le portant à reflux dans de l'acide chlorhydrique 6N. Le mélange est ensuite concentré et alcalinisé dans 20 ml d'eau et 10 ml de soude 1N puis extrait au dichlorométhane. Un solide blanc est obtenu par concentration de la phase organique (1,08 g).

### Stade 2 : N{4-[3-(hexahydrocyclopenta[c]pyrrol-2(1H)-yl)propoxy]phényl}-4-fluorobenzamide

0,24 g (1mM) du composé synthétisé au stade précédent est dissout dans 2,5 ml de tétrahydrofurane anhydre puis refroidi dans un bain de glace. Sont ajoutés goutte à goutte successivement 0,21 ml (1,5 mM) de triéthylamine et 0,26 g (1 mM) de chlorure de 4-fluorobenzoyle. Le mélange est maintenu sous agitation dans un bain de glace, puis laissé à température ambiante sous agitation 16 h. La solution est diluée avec de l'acétate d'éthyle et extraite à la soude (6N), lavée à l'eau puis séchée sur sulfate de magnésium et concentrée. Le produit du titre peut être obtenu sous forme d'oxalate par recristallisation dans l'éthanol (voir stade 2 de l'exemple 18).

| *Microanalyses élémentaires:* | | | |
|---|---|---|---|
| | *C%* | *H%* | *N%* |
| *Calculé :* | *72,23* | *7,12* | *7,32* |
| *Trouvé :* | *72, 26* | *7,10* | *7, 34* |

### EXEMPLE 32 : N-{4-[3-(Hexahydrocyclopenta[c]pyrrol-2(1H)-yl)propoxylphényl}-2-fluorobenzamide

Le procédé expérimental est identique à celui de l'Exemple 31 en remplaçant au stade 2 le chlorure de 4-fluorobenzoyle, par le chlorure de 2-fluorobenzoyle.

| *Microanalyses élémentaires :* | | | |
|---|---|---|---|
| | *C%* | *H%* | *N%* |
| *Calculé :* | *72,23* | *7,12* | *7,32* |
| *Trouvé:* | *72,01* | *7, 03* | *7, 28* |

### EXEMPLE 33 : N-{4-(3-(Hexahydrocyclopenta[c]pyrrol-2(1H)propoxy]phényl}-2,4-difluorobenzamide

Le procédé expérimental est identique à celui de l'Exemple 31 en remplaçant au stade 2 le chlorure de 4-fluorobenzoyle, par le chlorure de 2,4-difluorobenzoyle.

| *Microanalyses élémentaires :* | | | |
|---|---|---|---|
| | *C%* | *H%* | *N%* |
| *Calculé :* | *68,98* | *6,54* | *7,00* |
| *Trouvé:* | *69, 02* | *6, 72* | *6, 99* |

### EXEMPLE 34 : N-{4-[3-(hexahydrocyclopenta[c]pyrrol-2(1H)-yl)propoxy]phényl}-4-trifluorométhylbenzamide

Le procédé expérimental est identique à celui de l'Exemple 31 en remplaçant au stade 2 le chlorure de 4-fluorobenzoyle, par le chlorure de 4-trifluorométhylbenzoyle.

| *Microanalyses élémentaires :* | | | |
|---|---|---|---|
| | *C%* | *H%* | *N%* |
| *Calculé :* | *66,65* | *6,29* | *6,48* |
| *Trouvé:* | *66,64* | *6,39* | *6,51* |

### EXEMPLE 35 : N-{4-[3-(Hexahydrocyclopenta[c]pyrrol-2(1H)-yl)propoxy]phényl}-2-trifluorométhylbenzamide

Le procédé expérimental est identique à celui de l'Exemple 31 en remplaçant au stade 2 le chlorure de 4-fluorobenzoyle, par le chlorure de 2-trifluorométhylbenzoyle.

| *Microanalyses élémentaires :* | | | |
|---|---|---|---|
| | *C%* | *H%* | *N%* |
| *Calculé :* | *66,65* | *6,29* | *6,48* |
| *Trouvé :* | *66,36* | *6,34* | *6,36* |

### EXEMPLE 36 : Oxalate du N-{4-[3-(hexahydrocyclopenta[c]pyrrol-2(1H)-yl)propoxy]phényl}-4-méthoxybenzamide

Le procédé expérimental est identique à celui de l'Exemple 31 en remplaçant au stade 2 le chlorure de 4-fluorobenzoyle, par le chlorure de 4-méthoxybenzoyle.

| *Microanalyses élémentaires :* | | | |
|---|---|---|---|
| | *C%* | *H%* | *N%* |
| *Calculé :* | *64,45* | *6,66* | *5,78* |
| *Trouvé :* | *64,57* | *6,65* | *5,78* |

### EXEMPLE 37 : N-{4-[3-(Hexahydrocyclopenta[c]pyrrol-2(1H)-yl)propoxylphényl}-2-naphtamide

Le procédé expérimental est identique à celui de l'Exemple 31 en remplaçant au stade 2 le chlorure de 4-fluorobenzoyle, par le chlorure de 2-naphtoyle.

| *Microanalyses élémentaires :* | | | |
|---|---|---|---|
| | *C%* | *H%* | *N%* |
| *Calculé :* | *78,23* | *7,29* | *6,76* |
| *Trouvé :* | *78,36* | *7,26* | *6,81* |

### EXEMPLE 38 : Oxalate du N-{4-[3-(hexahydrocyclopenta[c]pyrrol-2(1H)-yl)propoxylphényl}-1-naphtamide

Le procédé expérimental est identique à celui de l'Exemple 31 en remplaçant au stade 2 le chlorure de 4-fluorobenzoyle, par le chlorure de 1-naphtoyle.

| *Microanalyses élémentaires :* | | | |
|---|---|---|---|
| | *C%* | *H%* | *N%* |
| *Calculé :* | *69,03* | *6,39* | *5,55* |
| *Trouvé :* | *68,57* | *6,33* | *5,68* |

### EXEMPLE 39 : N-{4-[3-(Hexahydrocyctopenta[c]pyrrol-2(1H)-yl)propoxy]phényl}-2-furanecarboxamide

Le procédé expérimental est identique à celui de l'Exemple 31 en remplaçant au stade 2 le chlorure de 4-fluorobenzoyle, par le chlorure de 2-furoyle.

| *Microanalyses élémentaires :* | | | |
|---|---|---|---|
| | *C%* | *H%* | *N%* |
| *Calculé :* | *71,16* | *7,39* | *7,90* |
| *Trouvé :* | *70,90* | *7,44* | *7,87* |

### EXEMPLE 40 : N-{4-[3-(Hexahydrocyclopenta[c]pyrrol-2(1H)-yl)propoxy]phényl}-2-thiophènecarboxamide

Le procédé expérimental est identique à celui de l'Exemple 31 en remplaçant au stade 2 le chlorure de 4-fluorobenzoyle, par le chlorure de 2-thénoyle.

| *Microanalyses élémentaires*: | | | | |
|---|---|---|---|---|
| | *C%* | *H%* | *N%* | *S%* |
| *Calculé :* | *68,08* | *7,07* | *7,56* | *8,65* |
| *Trouvé :* | *68,21* | *7,09* | *7,50* | *8,52* |

### EXEMPLE 41 : N-{4-[3-(Hexahydrocyclopenta[c]pyrrol-2(1H)-yl)propoxy]-phényl}isonicotinamide

Le procédé expérimental est identique à celui de l'Exemple 31 en remplaçant au stade 2 le chlorure de 4-fluorobenzoyle, par le chlorure de l'isonicotinoyle.

| *Microanalyses élémentaires :* | | | |
|---|---|---|---|
| | *C%* | *H%* | *N%* |
| *Calculé :* | *72,30* | *7,45* | *11,50* |
| *Trouvé :* | *72,63* | *7,57* | *11,44* |

### EXEMPLE 42 : N-{4-[3-(Hexahydrocyclopenta[c]pyrrol-2(1H)-yl)propoxy]phényl}-benzo[b]thiophène-3-carboxamide

Le procédé expérimental est identique à celui de l'Exemple 31 en remplaçant au stade 2 le chlorure de 4-fluorobenzoyle, par le chlorure du benzo[b]thiophène-3-carbonyle.

| *Microanalyses élémentaires :* | | | | |
|---|---|---|---|---|
| | *C%* | *H%* | *N%* | *S%* |
| *Calculé :* | *71,40* | *6,71* | *6,66* | *7,62* |
| *Trouvé:* | *71,00* | *6,89* | *6,57* | *7,41* |

### EXEMPLE 43 : N-{4-[3-(Hexahydrocyclopenta[c]pyrrol-2(1H)-yl)propoxy]phényl}-2-phénylacétamide

Le procédé expérimental est identique à celui de l'Exemple 31 en remplaçant au stade 2 le chlorure de 4-fluorobenzoyle, par le chlorure de phénylacétyle.

| *Microanalyses élémentaires :* | | | |
|---|---|---|---|
| | *C%* | *H%* | *N%* |
| *Calculé :* | *76,16* | *7,99* | *7,40* |
| *Trouvé:* | *76,33* | *8,00* | *7,26* |

### EXEMPLE 44 : Oxalate du N-{4-[3-(hexahydrocyclopenta[c]gyrrol-2(1H)-yl)piropoxy]phényl}-2-(3,4-diméthoxyphényl)acétamide

Le procédé expérimental est identique à celui de l'Exemple 31 en remplaçant au stade 2 le chlorure de 4-fluorobenzoyle, par le chlorure de (3,4-diméthoxyphényl)acétyle.

| *Microanalyses élémentaires :* | | | |
|---|---|---|---|
| | *C%* | *H%* | *N%* |
| *Calculé :* | *63,62* | *6,86* | *5,30* |
| *Trouvé:* | *63,32* | *6,72* | *5,22* |

### EXEMPLE 45 : N-{4-[3-(Hexahydrocyclopenta[c]pyrrol-2(1H)-yl)propoxy]phényl}-2-(2-thiényl)acétamide

Le procédé expérimental est identique à celui de l'Exemple 31 en remplaçant au stade 2 le chlorure de 4-fluorobenzoyle, par le chlorure de (2-thiényl)acétyle.

| *Microanalyses élémentaires :* | | | | |
|---|---|---|---|---|
| | *C%* | *H%* | *N%* | *S%* |
| *Calculé :* | *68,72* | *7,34* | *7,28* | *8,34* |
| *Trouvé :* | *68,57* | *7,45* | *7,20* | *8,92* |

### EXEMPLE 46 : Oxalate du N-{4-[3-(hexahydrocyclopenta[c]pyrrol-2(1H)-yl)propoxy]phényl}-2,2-diphénylacétamide

Le procédé expérimental est identique à celui de l'Exemple 31 en remplaçant au stade 2 le chlorure de 4-fluorobenzoyle, par le chlorure de diphénylacétyle.

| *Microanalyses élémentaires :* | | | |
|---|---|---|---|
| | *C%* | *H%* | *N%* |
| *Calculé :* | *70,57* | *6,66* | *5,14* |
| *Trouvé:* | *70,15* | *6, 72* | *5,18* |

### EXEMPLE 47 : Oxalate du N-{4-[3-(hexahydrocyclopenta[c]pyrrol-2(1H)-yl)propoxy]phényl}-3-phénylpropanamide

Le procédé expérimental est identique à celui de l'Exemple 31 en remplaçant au stade 2 le chlorure de 4-fluorobenzoyle, par le chlorure de 3-phénylpropanoyle.

| *Microanalyses élémentaires :* | | | |
|---|---|---|---|
| | *C%* | *H%* | *N%* |
| *Calculé :* | *67,20* | *7,10* | *5,80* |
| *Trouvé:* | *66,85* | *7,14* | *5,74* |

### EXEMPLE 48 : Oxalate du N-{4-[3-(hexahydrocyclopenta[c]pyrrol-2(1H)-yl)propoxy]phényl}-2-méthoxyacétamide

Le procédé expérimental est identique à celui de l'Exemple 31 en remplaçant au stade 2 le chlorure de 4-fluorobenzoyle, par le chlorure de méthoxyacétyle.

| *Microanalyses élémentaires :* | | | |
|---|---|---|---|
| | *C%* | *H%* | *N%* |
| *Calculé :* | *56,96* | *6,98* | *6,09* |
| *Trouvé :* | *57,28* | *6,77* | *6,05* |

### EXEMPLE 49 : N'-{4-[3-(Hexahydrocyclopenta[c]pyrrol-2(1H)-yl)propoxy]phényl}-N,N-diméthylurée

Le procédé expérimental est identique à celui de l'Exemple 31 en remplaçant au stade 2 le chlorure de 4-fluorobenzoyle, par le chlorure de diméthylcarbamoyle.

| *Microanalyses élémentaires :* | | | |
|---|---|---|---|
| | *C%* | *H%* | *N%* |
| *Calculé :* | *68,85* | *8,82* | *12,68* |
| *Trouvé :* | *68,84* | *9,09* | *12,29* |

### EXEMPLE 50 : N-{4-[3-(Hexahydrocyclopenta[c]pyrrol-2(1H)-yl)propoxy]phényl}-morpholinocarboxamide

Le procédé expérimental est identique à celui de l'Exemple 31 en remplaçant au stade 2 le chlorure de 4-fluorobenzoyle, par le chlorure de morpholinocarbonyle.

| *Microanalyses élémentaires :* | | | |
|---|---|---|---|
| | *C%* | *H%* | *N%* |
| *Calculé :* | *67,53* | *8,37* | *11,25* |
| *Trouvé :* | *67, 67* | *8, 67* | *11,41* |

### EXEMPLE 51 : 4-[3-(Hexahydrocyclopenta[c]pyrrol-2(1H)-yl)propoxy]-N-phénylbenzamide

Le procédé expérimental est identique à celui de l'Exemple 1 en remplaçant au stade 1 le 4-hydroxybenzonitrile, par le 4-hydroxy-*N*-phénylbenzamide.

| *Microanalyses élémentaires :* | | | |
|---|---|---|---|
| | *C%* | *H%* | *N%* |
| *Calculé :* | *75,79* | *7,74* | *7,69* |
| *Trouvé :* | *75,46* | *7,82* | *7,60* |

### EXEMPLE 52 ; 4-[3-(Hexahydrocyclopenta[c]pyrrol-2(1H)-yl)propoxy]-N-(4-fluorophényl)benzamide

Le procédé expérimental est identique à celui de l'Exemple 1 en remplaçant au stade 1 le 4-hydroxybenzonitrile, par le *N*-(4-fluorophényl)-4-hydroxybenzamide.

| *Microanalyses élémentaires :* | | | |
|---|---|---|---|
| | *C%* | *H%* | *N%* |
| *Calculé :* | *72,23* | *7,12* | *7,32* |
| *Trouvé :* | *71,85* | *7,23* | *7,31* |

### EXEMPLE 53 : 4-[3-(Hexahydrocyclopenta[c]pyrrol-2(1H)-yl)progoxy]-N-(1,3-benzodioxol-5-yl)benzamide

Le procédé expérimental est identique à celui de l'Exemple 1 en remplaçant au stade 1 le 4-hydroxybenzonitrile, par le N-(1,3-benzodioxol-5-yl)-4-hydroxybenzamide.

| *Microanalyses élémentaires :* | | | |
|---|---|---|---|
| | *C%* | *H%* | *N%* |
| *Calculé:* | *70,57* | *6,91* | *6,86* |
| *Trouvé :* | *70, 46* | *7, 06* | *7, 08* |

### EXEMPLE 54 : 4-[3-(Hexahydrocyclopenta[c]pyrrol-2(1H)-yl)propoxy]-N-cyclohexylbenzamide

Le procédé expérimental est identique à celui de l'Exemple 1 en remplaçant au stade 1 le 4-hydroxybenzonitrile, par le *N*-cyclohexyl-4-hydroxybenzamide.

| *Microanalyses élémentaires :* | | | |
|---|---|---|---|
| | *C%* | *H%* | *N%* |
| *Calculé :* | *74, 56* | *9, 25* | *7, 56* |
| *Trouvé :* | *74,11* | *9, 30* | *7, 36* |

### EXEMPLE 55 : Oxalate du 4-[3-(hexahydrocyctopenta[c]pyrrol-2(1H)-yl)propoxy]-N-méthyl-N-cyclohexylbenzamide

Le procédé expérimental est identique à celui de l'Exemple 1 en remplaçant au stade 1 le 4-hydroxybenzonitrile, par le *N*-méthyl-*N*-cyclohexyl-4-hydroxybenzamide.

| *Microanalyses élémentaires:* | | | |
|---|---|---|---|
| | *C %* | *H%* | *N%* |
| *Calculé :* | *65,80* | *8,07* | *5,90* |
| *Trouvé :* | *65,06* | *7,64* | *6,07* |

### EXEMPLE 56 : 4-[3-(Hexahydrocyclopenta[c]pyrrol-2(1H)-yl)propoxy]-N,N-dicyclo-hexylbenzamide

Le procédé expérimental est identique à celui de l'Exemple 1 en remplaçant au stade 1 le 4-hydroxybenzonitrile, par la *N,N-*dicydohexyl-4-hydroxybenzamide.

| *Microanalyses élémentaires :* | | | |
|---|---|---|---|
| | *C %* | *H%* | *N%* |
| *Calculé:* | *76,95* | *9,80* | *6,19* |
| *Trouvé :* | *76,23* | *9,86* | *6,11* |

### EXEMPLE 57 : Oxalate du 2-[3-(4-pipéridinocarbonylphénoxy)propyl]-octahydrocyclopenta[c]pyrrole

Le procédé expérimental est identique à celui de l'Exemple 1 en remplaçant au stade 1 le 4-hydroxybenzonitrile, par le 4-pipéridinocarbonylphénol.

| *Microanalyses élémentaires :* | | | |
|---|---|---|---|
| | *C %* | *H%* | *N%* |
| *Calculé :* | *61,09* | *7,18* | *5,70* |
| *Trouvé :* | *61,05* | *7,33* | *5,60* |

### EXEMPLE 58 ; Dioxalate de la 1-{4-[3-(hexahydrocyclopenta[c]pyrrol-2(1H)-yl)propoxy]benzoyl}-1,2,3,4-tétrahydroquinoléine

Le procédé expérimental est identique à celui de l'Exemple 1 en remplaçant au stade 1 le 4-hydroxybenzonitrile, par le 4-(3,4-dihydro-1(2*H*)-quinolylcarbonyl)phénol.

| *Microanalyses élémentaires :* | | | |
|---|---|---|---|
| | *C%* | *H%* | *N%* |
| *Calculé :* | *63,33* | *6,40* | *5,72* |
| *Trouvé :* | *63,47* | *6,61* | *5,16* |

### EXEMPLE 59 : Dioxalate du 2-[3-(4-[pipéridinopipéridinocarbonyl]-phénoxy)propyl]octahydrocyclopenta[c]pyrrole

Le procédé expérimental est identique à celui de l'Exemple 1 en remplaçant au stade 1 le 4-hydroxybenzonitrile, par le 4-pipéridinopipéridinocarbonylphénol.

| *Microanalyses élémentaires :* | | | |
|---|---|---|---|
| | *C%* | *H%* | *N%* |
| *Calculé :* | *57,82* | *6,98* | *6,32* |
| *Trouvé:* | *57,51* | *7,17* | *6,27* |

### EXEMPLE 60 : Dioxalate du 2-(3-{4-[(4-méthyl-1-pipérazinyl)carbonyl]-phénoxy}propyl)octahydrocyclopenta[c]pyrrole

Le procédé expérimental est identique à celui de l'Exemple 1 en remplaçant au stade 1 le 4-hydroxybenzonitrile, par le 4-[(4-méthyl-1-pipérazinyl)carbonyl)phénol.

| *Microanalyses élémentaires :* | | | |
|---|---|---|---|
| | *C%* | *H%* | *N%* |
| *Calculé :* | *54,45* | *6,26* | *7,06* |
| *Trouvé :* | *54,82* | *6,51* | *7,00* |

### EXEMPLE 61 : Dioxalate du 2-(3-{4-[(4-benzyl-1-pipérazinyl)carbonyl]-phénoxy}propyl)octahydrocyclopenta[c]pyrrole

Le procédé expérimental est identique à celui de l'Exemple 1 en remplaçant au stade 1 le 4-hydroxybenzonitrile, par le 4-[(4-benzyl-1-pipérazinyl)carbonyl]phénol.

| *Microanalyses élémentaires :* | | | |
|---|---|---|---|
| | *C%* | *H%* | *N%* |
| *Calculé :* | *56,90* | *6,04* | *5,85* |
| *Trouvé :* | *58,55* | *6,39* | *6,43* |

### EXEMPLE 62 : 4-[3-(Hexahydrocyclopenta[c]pyrrol-2(1H)-yl)propoxy]-N-(1-benzyl-pipéridino)benzamide

Le procédé expérimental est identique à celui de l'Exemple 1 en remplaçant au stade 1 le 4-hydroxybenzonitrile, par le *N*-(1-benzylpipéridino)-4-hydroxybenzamide.

| *Microanalyses élémentaires :* | | | |
|---|---|---|---|
| | *C%* | *H%* | *N%* |
| *Calculé :* | *75,45* | *8,52* | *9,10* |
| *Trouvé :* | *75,36* | *8,52* | *9,07* |

### EXEMPLE 63 : 4-[3-(Hexahydrocyclapenta[c]pyrrol-2(1H)propoxy]-N-(cyclopropylméthyl)benzamide

Le procédé expérimental est identique à celui de l'Exemple 1 en remplaçant au stade 1 le 4-hydroxybenzonitrile, par le *N*-(cyaopropylméthyl)-4-hydroxybenzamide.

| *Microanalyses élémentaires :* | | | |
|---|---|---|---|
| | *C%* | *H%* | *N%* |
| *Calculé :* | *73,65* | *8,83* | *8,18* |
| *Trouvé :* | *72,99* | *8,92* | *8,80* |

### EXEMPLE 64: 4-[3-(Hexahydrocyclopenta[c]pyrrol-2(1H)-yl)propoxy]-N-benzylbenzamide

Le procédé expérimental est identique à celui de l'Exemple 1 en remplaçant au stade 1 le 4-hydroxybenzonitrile, par le N-benzyl-4-hydroxybenzamide.

| *Microanalyses élémentaires :* | | | |
|---|---|---|---|
| | *C%* | *H%* | *N%* |
| *Calculé :* | *76,16* | *7,99* | *7,40* |
| *Trouvé :* | *76, 20* | *8, 06* | *7,41* |

### EXEMPLE 65: Oxalate du 4-[3-(hexabydrocyclopenta[c]pyrrol-2(1H)-yl)propoxy]-N-benzyl-N-méthylbenzamide

Le procédé expérimental est identique à celui de l'Exemple 1 en remplaçant au stade 1 le 4-hydroxybenzonitrile, par le N-benzyl-N-méthyl-4-hydroxybenzamide.

| *Microanalyses élémentaires :* | | | |
|---|---|---|---|
| | *C%* | *H%* | *N%* |
| *Calculé :* | *67,20* | *7,10* | *5,80* |
| *Trouvé:* | *66,85* | *7,17* | *5,82* |

### EXEMPLE 66 : Oxalate du 4-[3-(hexahydrocyclopenta[c]pyrrol-2(1H)-yl)propoxyl-N-benzyl-N-(4-méthoxyphényl)benzamide

Le procédé expérimental est identique à celui de l'Exemple 1 en remplaçant au stade 1 le 4-hydroxybenzonitrile, par le *N*-benzyl-*N*-(4-méthoxyphényl)-4-hydroxybenzamide.

| *Microanalyses élémentaires:* | | | |
|---|---|---|---|
| | *C%* | *H%* | *N%* |
| *Calculé :* | *68,97* | *6,66* | *4,87* |
| *Trouvé :* | *68,18* | *6,50* | *4,86* |

### EXEMPLE 67 : 4-[3-(Hexahydrocyclopenta[c]pyrrol-2(1H)-yl)propoxy]-N-(4-méthylbenzyl)benzamide

Le procédé expérimental est identique à celui de l'Exemple 1 en remplaçant au stade 1 le 4-hydroxybenzonitrile, par le *N*-(4-méthylbenzyl)-4-hydroxybenzamide.

| *Microanalyses élémentaires :* | | | |
|---|---|---|---|
| | *C%* | *H%* | *N%* |
| *Calculé :* | *76,50* | *8,22* | *7,14* |
| *Trouvé :* | *76,28* | *8,19* | *7,06* |

### EXEMPLE 68 : 4[3-(Hexahydrocyclopenta[c]pyrrol-2(1H)-yl)propoxy]-N-(3 méthylbenzyl)benzamide

Le procédé expérimental est identique à celui de l'Exemple 1 en remplaçant au stade 1 le 4-hydroxybenzonitrile, par le *N*-(3-méthylbenzyl)-4-hydroxybenzamide.

| *Microanalyses élémentaires :* | | | |
|---|---|---|---|
| | *C%* | *H%* | *N%* |
| *Calculé:* | *76,50* | *8,22* | *7,14* |
| *Trouvé:* | *76,01* | *8,31* | *6,96* |

### EXEMPLE 69 : 4-[3-(Hexahydrocyclopenta[c]pyrrol-2(1H)-yl)propoxy]-N-(2-méthylbenzyl)benzamide

Le procédé expérimental est identique à celui de l'Exemple 1 en remplaçant au stade 1 le 4-hydroxybenzonitrile, par le *N*-(2-méthylbenzyl)-4-hydroxybenzamide.

| *Microanalyses élémentaires :* | | | |
|---|---|---|---|
| | *C%* | *H%* | *N%* |
| *Calculé :* | *76,50* | *8,22* | *7,14* |
| *Trouvé :* | *76, 38* | *8, 32* | *7,05* |

### EXEMPLE 70 : 4-[3-(Hexahydrocyclopenta[c]pyrrol-2(1H)-yl)propoxy]-N-(4-trifluorométhylbenzyl)benzamide

Le procédé expérimental est identique à celui de l'Exemple 1 en remplaçant au stade 1 le 4-hydroxybenzonitrile, par le *N*-(4-trifluorométhylbenzyl)-4-hydroxybenzamide.

| *Microanalyses élémentaires :* | | | |
|---|---|---|---|
| | *C%* | *H%* | *N%* |
| *Calculé :* | *67,25* | *6,55* | *6,27* |
| *Trouvé :* | *67,24* | *6,47* | *6,23* |

### EXEMPLE 71 : Oxalate du 4-[3-(hexahydrocyclopenta[c]pyrrol-2(1H)-yl)propoxy]-N-(3-trifluorométhylbenzyl)benzamide

Le procédé expérimental est identique à celui de l'Exemple 1 en remplaçant au stade 1 le 4-hydroxybenzonitrile, par le *N*-(3-trifluorométhylbenzyl)-4-hydroxybenzamide.

| *Microanalyses élémentaires :* | | | |
|---|---|---|---|
| | *C%* | *H%* | *N%* |
| *Calculé :* | *60,44* | *5,82* | *5,22* |
| *Trouvé :* | *59,40* | *5,77* | *5,07* |

### EXEMPLE 72 : 4-[3-(Hexabydrocyclopenta[c]pyrrol-2(1H)-yl)propoxy]-N-(4-pyridylméthyl)benzamide

Le procédé expérimental est identique à celui de l'Exemple 1 en remplaçant au stade 1 le 4-hydroxybenzonitrile, par le *N*-(4-pyridylméthyl)-4-hydroxybenzamide.

| *Microanalyses élémentaires :* | | | |
|---|---|---|---|
| | *C%* | *H%* | *N%* |
| *Calculé :* | *72,79* | *7,70* | *11,07* |
| *Trouvé :* | *72,11* | *7,56* | *10,81* |

### EXEMPLE 73 : 4-[3-(Hexabydrocyclopenta[c]pyrrol-2(1H)-yl)propoxy]-N-furfurylbenzamide

Le procédé expérimental est identique à celui de l'Exemple 1 en remplaçant au stade 1 le 4-hydroxybenzonitrile, par le N-farfuryl-4-hydroxybenzamide.

| *Microanalyses élémentaires :* | | | |
|---|---|---|---|
| | *C%* | *H%* | *N%* |
| *Calculé :* | *71,71* | *7,66* | *7,60* |
| *Trouvé:* | *70,68* | *7,77* | *7,56* |

### EXEMPLE 74 : 4-(3-(Hexahydrocyclopenta[c]pyrrol-2(1H)-yl)propoxy]-N-[2-(2-thiényl)éthyl]benzamide

Le procédé expérimental est identique à celui de l'Exemple 1 en remplaçant au stade 1 le 4-hydroxybenzonitrile, par le *N*-[2-(2-thiényhnéthyl)éthyl]-4-hydroxybenzamide.

| *Microanalyses élémentaires :* | | | | |
|---|---|---|---|---|
| | *C%* | *H%* | *N%* | *S%* |
| *Calculé :* | *69,31* | *7,59* | *7,03* | *8,05* |
| *Trouvé :* | *69,28* | *7,63* | *6,89* | *8,01* |

### EXEMPLE 75 : 4-[3-(Hexahydrocyclopenta[c]pyrrol-2(1H)-yl)propoxy]-N-(3,4-diméthoxyphénéthyl)benzamide

Le procédé expérimental est identique à celui de l'Exemple 1 en remplaçant au stade 1 le 4-hydroxybenzonitrile, par le *N*-(3,4-diméthoxyphénéthyl)-4-hydroxybenzamide.

| *Microanalyses élémentaires :* | | | |
|---|---|---|---|
| | *C%* | *H%* | *N%* |
| *Calculé :* | *71,65* | *8,02* | *6,19* |
| *Trouvé:* | *71,80* | *8,09* | *6,16* |

### EXEMPLE 76 : 4-[3-(Hexahydrocyclopenta[c]pyrrol-2(1H)-yl)propoxy]-N-(2-pipéridinoéthyl)benzamide

Le procédé expérimental est identique à celui de l'Exemple 1 en remplaçant au stade 1 le 4-hydroxybenzonitrile, par le N-(2-pipéridinoéthyl)-4-hydroxybenzamide.

| | |
|---|---|
| RMN¹H (DMSO D₆): δ (ppm): | 1,20-1,75 (m, 12*H*); 1,90 (quint, 2*H*); 2,15 (m, 2*H*); 2,30-2,50 (m, 8*H*); 2,60 (m, 4*H*); 3,55 (quad, 2*H*); 4,05 (t,2*H*) ; 7,00 (d,2*H*); 7,80 (d,2*H*); 8,20 (t,1*H*). |

### EXEMPLE 77 ; 4-[3-(Hexahydrocyclopenta[c]pyrrol-2(1H)-yl)propoxy]-N-(2-morpholinoéthyl)benzamide

Le procédé expérimental est identique à celui de l'Exemple 1 en remplaçant au stade 1 le 4-hydroxybenzonitrile, par le *N*-(2-morpholmoéthyl)-4-hydroxybenzamide.

| *Microanalyses élémentaires :* | | | |
|---|---|---|---|
| | *C%* | *H%* | *N%* |
| *Calculé :* | *68, 80* | *8, 79* | *10,46* |
| *Trouvé :* | *68, 62* | *8, 84* | *10,34* |

### EXEMPLE 78 : Dioxalate du 4-[3-(hexahydrocyclopenta[c]pyrrol-2(1H)-yl)propoxy]-N-[3-(1H-imidazol-1-yl)propyl]benzamide

Le procédé expérimental est identique à celui de l'Exemple 1 en remplaçant au stade 1 le 4-hydroxybenzonitrile, par le *N*-[3-(1H imidazol-1-yl)propyl]-4-hydroxybenzamide.

| *Microanalyses élémentaires :* | | | |
|---|---|---|---|
| | *C%* | *H%* | *N%* |
| *Calculé :* | *56,24* | *6,29* | *9,72* |
| *Trouvé :* | *55,99* | *6, 44* | *9, 60* |

### EXEMPLE 79 : 4-[3-(Hexahydrocyclopenta[c]pyrrol-2(1H)-yl)propoxy]-N-(2-phénoxyéthyl)benzamide

Le procédé expérimental est identique à celui de l'Exemple 1 en remplaçant au stade 1 le 4-hydroxybenzonitrile, par le *N*-(2-phénoxyéthyl)-4-hydroxybenzamide.

| *Microanalyses élémentaires :* | | | |
|---|---|---|---|
| | *C%* | *H%* | *N%* |
| *Calculé :* | *73,50* | *7,89* | *6,86* |
| *Trouvé:* | *72,76* | *7,82* | *6,85* |

### EXEMPLE 80 : Oxalate du 4-[3-(hexahydrocyclopenta[c]pyrrol-2(1H)-yl)propoxy]-N-(2-méthoxyéthyl)benzamide

Le procédé expérimental est identique à celui de l'Exemple 1 en remplaçant au stade 1 le 4-hydroxybenzonitrile, par le *N*-(2-méthoxyéthyl)-4-hydroxybenzamide.

| *Microanalyses élémentaires :* | | | |
|---|---|---|---|
| | *C%* | *H%* | *N%* |
| *Calculé :* | *60,54* | *7,39* | *6,42* |
| *Trouvé :* | *61,07* | *7,54* | *6,49* |

### EXEMPLE 81 : Oxalate du 4-[3-(hexahydrocyclopenta[c]pyrrol-2(1H)-yl)propoxy]-N-[2-méthoxy-1-(méthoxyméthyl)éthyl]benzamide

Le procédé expérimental est identique à celui de l'Exemple 1 en remplaçant au stade 1 le 4-hydroxybenzonitrile, par le *N*-[2-méthoxy-1-(méthoxyméthyl)éthyl]-4-hydroxy-benzamide.

| *Microanalyses élémentaires :* | | | |
|---|---|---|---|
| | *C%* | *H%* | *N%* |
| *Calculé :* | *59,99* | *7,55* | *5,83* |
| *Trouvé :* | *59,54* | *7,44* | *5,60* |

### EXEMPLE 82 : 4-[3-(Hexahydrocyclopenta[c]pyrrol-2(1H)-yl)propoxy]-N-(tert-butoxy)benzamide

Le procédé expérimental est identique à celui de l'Exemple 1 en remplaçant au stade 1 le 4-hydroxybenzonitrile, par le *N*-(*tert*-butoxy)-4-hydroxybenzamide.

| *Microanalyses élémentaires :* | | | |
|---|---|---|---|
| | *C%* | *H%* | *N%* |
| *Calculé :* | *69,97* | *8,95* | *7,77* |
| *Trouvé :* | *70,05* | *9,00* | *7,69* |

### EXEMPLE 83 : Oxalate du 4-[3-(hexahydrocyclopenta[c]pyrrol-2(1H)-yl)propoxy]-N-(2-éthylbutyl)benzamide

Le procédé expérimental est identique à celui de l'Exemple 1 en remplaçant au stade 1 le 4-hydroxybenzonitrile, par le *N*-(2-éthylbutyl)-4-hydroxybenzamide.

| *Microanalyses élémentaires :* | | | |
|---|---|---|---|
| | *C%* | *H%* | *N%* |
| *Calculé :* | *64,91* | *8,28* | *6,06* |
| *Trouvé:* | *64,93* | *8,38* | *6,00* |

### EXEMPLE 84: 4-[3-(Hexabydrocyclopenta[c]pyrrol-2(1H)-yl)propoxy]-N-isopropylbenzamide

Le procédé expérimental est identique à celui de l'Exemple 1 en remplaçant au stade 1 le 4-hydroxybenzonitrile, par le N-isopropyl-4-hydroxybenzamide.

| *Microanalyses élémentaires :* | | | |
|---|---|---|---|
| | *C%* | *H%* | *N%* |
| *Calculé :* | *72,69* | *9,15* | *8,48* |
| *Trouvé:* | *73,10* | *9,36* | *8,54* |

### EXEMPLE 85 : Oxalate du 4-[3-(hexahydrocyclopenta[c]pyrrol-2(1H)-yl)propoxy]-N-(tert-butyl)benzamide

Le procédé expérimental est identique à celui de l'Exemple 1 en remplaçant au stade 1 le 4-hydroxybenzonitrile, par le N-(tert-butyl)-4-hydroxybenzamide.

| *Microanalyses élémentaires :* | | | |
|---|---|---|---|
| | *C%* | *H%* | *N%* |
| *Calculé :* | *63,57* | *7,89* | *6,45* |
| *Trouvé:* | *63, 82* | *8,12* | *6,32* |

### EXEMPLE 86 : Oxalate du 4-[3-(hexahydrocyclopenta[c]pyrrol-2(1H)-yl)propoxy]-N-propylbenzamide

Le procédé expérimental est identique à celui de l'Exemple 1 en remplaçant au stade 1 le 4-hydroxybenzonitrile, par le *N*-propyl-4-hydroxybenzamide.

| *Microanalyses élémentaires :* | | | |
|---|---|---|---|
| | *C%* | *H%* | *N%* |
| *Calculé :* | *62,84* | *7,67* | *6,66* |
| *Trouvé :* | *63,24* | *8,09* | *6,58* |

### EXEMPLE 87 : Oxalate du 4-[3-(hexahydrocyclopenta[c]pyrrol 2(1H)-yl)propoxy]-N,N-diméthylbenzamide

Le procédé expérimental est identique à celui de l'Exemple 1 en remplaçant au stade 1 le 4-hydroxybenzonitrile, par le *N,N-*diméthyl-4-hydroxybenzamide.

| *Microanalyses élémentaires :* | | | |
|---|---|---|---|
| | *C%* | *H%* | *N%* |
| *Calculé :* | *58,53* | *6,92* | *6,20* |
| *Trouvé :* | *58,51* | *6,99* | *6,09* |

### EXEMPLE 88 : Oxalate du 4-[3-(hexahydrocyclopenta[c]pyrrol-2(1H)-yl)propoxy]-N,N-dipropylbenzamide

Le procédé expérimental est identique à celui de l'Exemple 1 en remplaçant au stade 1 le 4-hydroxybenzonitrile, par le *N,N*-dipropyl-4-hydroxybenzamide.

| *Microanalyses élémentaires :* | | | |
|---|---|---|---|
| | *C%* | *H%* | *N%* |
| *Calculé :* | *64,91* | *8,28* | *6,06* |
| *Trouvé :* | *64,73* | *8,39* | *5,94* |

### EXEMPLE 89 : Oxalate du 4-[3-(hexahydrocyclopenta[c]pyrrol-2(1H)-yl)propoxy]-N-éthyl-N-méthylbenzamide

Le procédé expérimental est identique à celui de l'Exemple 1 en remplaçant au stade 1 le 4-hydroxybenzonitrile, par le *N*-éthyl-*N*-méthyl-4-hydroxybenzamide.

| *Microanalyses élémentaires :* | | | |
|---|---|---|---|
| | *C%* | *H%* | *N%* |
| *Calculé :* | *59, 34* | *7,15* | *6,02* |
| *Trouvé :* | *59,26* | *7,16* | *5,91* |

### EXEMPLE 90 : Oxalate du 4-[3-(hexahydrocyclopenta[c]pyrrol-2(1H)-yl)propoxy]-N-propyl-N-méthylbenzamide

Le procédé expérimental est identique à celui de l'Exemple 1 en remplaçant au stade 1 le 4-hydroxybenzonitrile, par le *N*-propyl-*N*-méthyl-4-hydroxybenzamide.

| *Microanalyses élémentaires :* | | | |
|---|---|---|---|
| | *C%* | *H%* | *N%* |
| *Calculé:* | *63,57* | *7,89* | *6,45* |
| *Trouvé :* | *63,62* | *8,11* | *6,38* |

### EXEMPLE 91 : Oxalate du 4-[3-(hexahydrocyclopenta[c]pyrrol-2(1H)-yl)propoxy]-N-isopropyl-N-méthylbenzamide

Le procédé expérimental est identique à celui de l'Exemple 1 en remplaçant au stade 1 le 4-hydroxybenzonitrile, par le *N*-isopropyl-*N*-méthyl-4-hydroxybenzamide.

| *Microanalyses élémentaires :* | | | |
|---|---|---|---|
| | *C%* | *H%* | *N%* |
| *Calculé :* | *63,57* | *7,89* | *6,45* |
| *Trouvé :* | *63,95* | *8,30* | *6,37* |

### EXEMPLE 92 : Oxalate du 4-[3-(hexahydrocyclopenta[c]pyrrol-2(1H)-yl)propoxy]-N-(tert-butyl)-N- méthylbenzamide

Le procédé expérimental est identique à celui de l'Exemple 1 en remplaçant au stade 1 le 4-hydroxybenzonitrile, par le *N*-(*tert*-butyl)-*N*-méthyl-4-hydroxybenzamide.

| *Microanalyses élémentaires :* | | | |
|---|---|---|---|
| | *C%* | *H%* | *N%* |
| *Calculé :* | *64,26* | *8,09* | *6,25* |
| *Trouvé:* | *63,81* | *8,10* | *6,20* |

### EXEMPLE 93 : :4-[3-(Hexahydrocyclopenta[c]pyrrol-2(1H)-yl)propoxy]-N-méthylbenzamide

Le procédé expérimental est identique à celui de l'Exemple 1 en remplaçant au stade 1 le 4-hydroxybenzonitrile, par le *N*-méthyl-4-hydroxybenzamide.

| *Microanalyses élémentaires :* | | | |
|---|---|---|---|
| | *C%* | *H%* | *N%* |
| *Calculé :* | *71,49* | *8,67* | *9,26* |
| *Trouvé :* | *71,35* | *8,85* | *9,18* |

### EXEMPLE 94 : Oxalate du 4-[3-(hexahydrocyclopenta[c]pyrrol-2(1H)-yl)propoxyl-3-bromobenzamide

Le procédé expérimental est identique à celui de l'Exemple 1 en remplaçant au stade 1 le 4-hydroxybenzonitrile, par le 4-hydroxy-3-bromobenzamide.
*ESI*⁺ *: [M*+*H]*⁺ *367,1031 (théorie : 367,1021)*

### EXEMPLE 95 : Dioxalate du 2-{3-[4-(1H-imidazol-1-yl)phénoxy]propyl}-octahydrocyclopenta[c]pyrrole

Le procédé expérimental est identique à celui de l'Exemple 1 en remplaçant au stade 1 le 4-hydroxybenzonitrile, par le 4-(1*H*-imidazol-1-yl)phénol.

| *Microanalyses élémentaires :* | | | |
|---|---|---|---|
| | *C%* | *H%* | *N%* |
| *Calculé :* | *55,68* | *5,88* | *8,40* |
| *Trouvé :* | *55,81* | *5,57* | *8,51* |

### EXEMPLE 96 : Oxalate du 2-{3-[4-(1H 1,2,4-triazol-1-yl)phénoxy]propyl}-octahydrocyclopenta[c] pyrrole

Le procédé expérimental est identique à celui de l'Exemple 1 en remplaçant au stade 1 le 4-hydroxybenzonitrile, par le 4-(1H 1,2,4-triazol-1-yl)phénol.

| *Microanalyses élémentaires :* | | | |
|---|---|---|---|
| | *C%* | *H%* | *N%* |
| *Calculé :* | *59,69* | *6,51* | *13,92* |
| *Trouvé :* | *58,83* | *6,39* | *13,35* |

### EXEMPLE 97 : Oxalate de la N-[4-(3-hexahydrocyclopenta[c]pyrrol-2(1H)-ylpropoxy)phényl]-N-(2-pyrimidyl)amine

Le procédé expérimental est identique à celui de l'Exemple 1 en remplaçant au stade 1 le 4-hydroxybenzonitrile, par le 4-(2-pyrimidylamino)phénol.

| *Microanalyses élémentaires :* | | | |
|---|---|---|---|
| | *C%* | *H%* | *N%* |
| *Calculé :* | *59,92* | *6,37* | *12,42* |
| *Trouvé:* | *59,43* | *6,47* | *11,67* |

### EXEMPLE 98 : Dioxalate de la N-[4-(3-hexahydrocyclopenta[c]pyrrol-2(1H)-ylpropoxy)phényl]-2-quinolylamine

Le procédé expérimental est identique à celui de l'Exemple 1 en remplaçant au stade 1 le 4-hydroxybenzonitrile, par le 4-(2-quinolylamino)phénol.

| *Microanalyses élémentaires :* | | | |
|---|---|---|---|
| | *C%* | *H%* | *N%* |
| *Calculé :* | *61,37* | *5,86* | *7,40* |
| *Trouvé :* | *61,49* | *6,02* | *7,32* |

### EXEMPLE 99 : N-[4-(3-Hexahydrocyclopenta[c]pyrrol-2(1H)-ylpropoxy)phényl]-1-isoquinolylamine

Le procédé expérimental est identique à celui de l'Exemple 1 en remplaçant au stade 1 le 4-hydroxybenzonitrile, par le 4-(1-isoquinolylamino)phénol.

| *Microanalyses élémentaires :* | | | |
|---|---|---|---|
| | *C%* | *H%* | *N%* |
| *Calculé :* | *77,49* | *7,54* | *10,84* |
| *Trouvé :* | *76,81* | *7,68* | *10,68* |

### EXEMPLE 100 : N-[4-(3-Hebahydrocyclapenta[e]pyrrol-2(1H)-ylpropoxy)phényl]-9H-purin-6-ylamine

Le procédé expérimental est identique à celui de l'Exemple 1 en remplaçant au stade 1 le 4-hydroxybenzonitrile, par le 4-(9*H*-purin-6-ylamino)phénol.

| *Microanalyses élémentaires :* | | | |
|---|---|---|---|
| | *C%* | *H%* | *N%* |
| *Calculé :* | *66,64* | *6,92* | *22,20* |
| *Trouvé:* | *66,84* | *7,03* | *21,81* |

### EXEMPLE 101 : Oxalate du 4-(3-octahydro-2(1H)-isoquinolinylpropoxy)benzonitrile

Le procédé expérimental est identique à celui de l'Exemple 1 en remplaçant au stade 2 l'octahydrocyclopenta[c]pyrrole par la décahydroisoquinoline. La décahydroisoquinoline a été synthétisée selon la méthode de Wiktop. B (J. Am. Chem. Soc., 1948, 70, 2617).

| *Microanalyses élémentaires :* | | | |
|---|---|---|---|
| | *C%* | *H%* | *N%* |
| *Calculé :* | *64,93* | *7,27* | *7,21* |
| *Trouvé :* | *63,93* | *7,03* | *7,01* |

### EXEMPLE 102 : Oxalate du 4-(3-octahydro-2H-isoindol-2-ylpropoxy)benzonitrile

Le procédé expérimental est identique à celui de l'Exemple 1 en remplaçant au stade 2 l'octahydrocyclopenta[c]pyrrole par l'octahydroisoindole. L'octahydroisoindole a été synthétisée selon la méthode de Matsuki et al. (Chem. Pharm. Bull., 1994, 42(1), 9-18).

| *Microanalyses élémentaires :* | | | |
|---|---|---|---|
| | *C%* | *H%* | *N%* |
| *Calculé :* | *64,15* | *7,00* | *7,48* |
| *Trouvé :* | *63, 86* | *6,89* | *7,55* |

### EXEMPLE 103 : Oxalate du 4-(4-octabydro-2(1H)-isoquinolinylbutoxy)benzonitrile

Le procédé expérimental est identique à celui de l'Exemple 3 en remplaçant au stade 2 l'octahydrocyclopenta[*c*]pyrrole par la décahydroisoquinoline.

| *Microanalyses élémentaires :* | | | |
|---|---|---|---|
| | *C%* | *H%* | *N%* |
| *Calculé :* | *63,98* | *7,29* | *6,66* |
| *Trouvé:* | *64,54* | *7,25* | *6,73* |

### EXEMPLE 104 : 4-(3-Octahydro-2H-isoindol-2-ylpropoaxy)benzamide

### Stade 1 : 4-(3-octahydro-2H-isoindol-2-ylpropoxy)benzonitrile

Le procédé expérimental est identique à celui de l'Exemple 1 en remplaçant au stade 2 l'octahydrocyclopenta[c]pyrrole par l'octahydroisoindole.

### Stade 2: 4-(3-octahydro-2H-isoindol-2-ylpropoxy)benzamide

Le procédé expérimental est identique à celui de l'Exemple 21 en remplaçant le composé de l'Exemple 18 par le composé du stade précédent.
*ESI*⁺ *: [M*+*H]*⁺ *303,2072 (théorie : 303,2073)*

### EXEMPLE 105 : N-Méthyl-4-(3-octahydro-2H-isoindol-2-ylpropoxy)benzamide

Le procédé expérimental est identique à celui de l'Exemple 1 en remplaçant au stade 1 le 4-hydroxybenzonitrile par le *N*-méthyl-4-hydroxybenzamide, et au stade 2 l'octahydrocyclopenta[c]pyrrole par l'octahydroisoindole.
*ESI*⁺ *: [M*+*H]*⁺ *317,2240 (théorie : 317,2229)*

### EXEMPLE 106 : N,N-Diméthyl-4-(3-octahydro-2H-isoindol-2-ylpropoxy)benzamide

Le procédé expérimental est identique à celui de l'Exemple 1 en remplaçant au stade 1 le 4-hydroxybenzonitrile par le *N,N*-diméthyl-4-hydroxybenzamide, et au stade 2 l'octahydro-cyclopenta[*c*]pyrrole par l'octahydroisoindole.

| *Microanalyses élémentaires :* | | | |
|---|---|---|---|
| | *C%* | *H%* | *N%* |
| *Calculé :* | *72, 69* | *9,15* | *8,48* |
| *Trouvé :* | *72,25* | *9,21* | *8,37* |

### ETUDES PHARMACOLOGIQUES DES COMPOSES DE L'INVENTION

### EXEMPLE A : Dosages cérébraux de la N^{t}-Méthylhistamine chez la souris NMRI

Cette étude réalisée selon la méthode de Taylor et al. (Biochem. Pharm., 1992, 44, 1261-1267), a pour objectif d'évaluer l'activité *ex vivo* des composés de la présente invention en tant qu'antagonistes des récepteurs histaminergiques centraux de type H3. Cette activité est révélée par la mesure, après traitement par voie orale des composés sous étude, des taux centraux de N^{t}-Méthylhistamine, métabolite principal de l'histamine. Une augmentation des concentrations cérébrales de N^{t}-Méthylhistamine signe une augmentation du turn-over de l'histamine par blocage des récepteurs histaminergiques centraux de type H3.

Des souris NMRI (18-20g) sont traitées par voie orale par les composés de la présente invention ou par leur véhicule (20 ml/kg). Deux heures après le traitement pharmacologique, les animaux sont sacrifiés, les cerveaux sont prélevés, congelés dans l'azote liquide, pesés et homogénéisés dans HCIO₄ 0,1N à 4°C. Les homogénats sont centrifugés (15000g, 17 min, 4°C). Les surnageants sont récupérés et aliquotés. Les aliquotes sont congelés dans l'azote liquide et stockés à -80°C jusqu'à leur analyse.

La détermination des taux cérébraux de N^{t}-Méthylhistamine est réalisée par électrophorèse capillaire couplée à la détection par fluorescence induite par laser *(*J. Chromatogr. A., 1996, 755, 99-115). Les taux tissulaires de N^{t}-Méthylhistamine sont exprimés en ng/g de cerveau frais. La comparaison des taux cérébraux de N^{t}-Méthylhistamine entre les animaux traités par le véhicule (témoins) et les animaux traités (n=5/groupe) par les composés de la présente invention est effectuée par une analyse de variance à un facteur suivie si nécessaire par une analyse complémentaire (test de Dunnett).

Les résultats montrent que les composés de la présente invention sont capables, pour des doses comprises entre 3 et 10 mg/kg PO, d'augmenter de plus de 50 % les concentrations cérébrales endogènes de Nt-Méthylhistamine. A titre indicatif, les composés des exemples 4, 22 et 93 pour des doses de 3 mg/kg PO, augmentent de 52 %, 33 % et 90 % respectivement les concentrations cérébrales endogènes de Nt-Méthylhistamine et, le composé des exemples 1 et 22, pour une dose de 10 mg/kg PO, augmentent de 92% et 85 % respectivement les concentrations cérébrales endogènes de Nt-Méthylhistamine. Ces résultats montrent que les composés de la présente invention sont de puissants activateurs des systèmes histaminergiques centraux, actifs par voie orale et d'une durée d'action au moins égale à plusieurs heures.

### EXEMPLE B : Compositions pharmaceutiques

Formule de préparation pour 1000 comprimés dosés à 100 mg :

| | |
|---|---|
| Composé de l'exemple 22 | 100 g |
| Hydroxypropylcellulose | 20 g |
| Polyvinylpyrrolidone | 20 g |
| Amidon de blé | 150 g |
| Lactose | 900 g |
| Stéarate de magnésium | 30 g |

## Revendications

1. Composés de formule (I) : 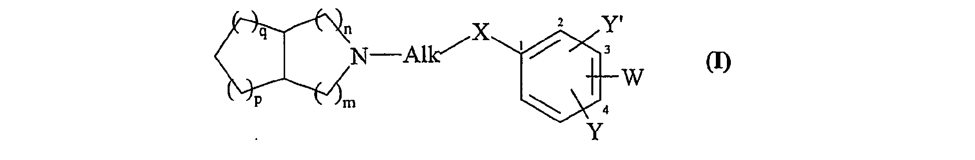 dans laquelle :
• **m** et **n**, identiques ou différents, représentent un entier compris inclusivement entre 0 et 2 avec la somme des deux entiers comprise inclusivement entre 2 et 3,
• **p** et **q,** identiques ou différents, représentent un entier compris inclusivement entre 0 et 2,
• **Alk** représente une chaîne alkylène, alkénylène ou alkynylène,
• **Y** et **Y**', identiques ou différents, représentent un atome d'hydrogène, d'halogène, ou un groupement alkyle, alkoxy, alkylthio, alkylsulfinyle, alkylsulfonyle, mercapto, hydroxy, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo ou cyano,
• X représente un atome d'oxygène, de soufre ou un groupement -N(R)-, avec R représentant un atome d'hydrogène ou un groupement alkyle,
• **W** représente un groupement choisi parmi cyano (lorsque X représente un atome d'oxygène ou un groupement -N(R)-), -N(R₁)-Z₁-R₂ et -Z₂-NR₁R₂, avec :
- Z₁ représentant -C(O)-, -C(S)-, -C(NR₄)-, *-C(O)-N(R₃)-, *-C(S)-N(R₃)-, *-C(NR₄)-N(R₃)-, *-C(O)O-, *-C(S)-O-, -S(O)ᵣ-, avec r = 1 ou 2, et * correspondant au point d'attachement à N(R₁),
- Z₂ représentant -C(O)-, -C(S)-, -C(NR₄)-, -S(O)ᵣ-, liaison,
- R₁, R₂, R₃ et R₄, identiques ou différents, représentant un atome d'hydrogène, un groupement alkyle éventuellement substitué, alkényle éventuellement substitué, alkynyle éventuellement substitué, alkoxy, cycloalkyle éventuellement substitué, un groupement hétérocycloalkyle éventuellement substitué, un groupement aryle éventuellement substitué ou hétéroaryle éventuellement substitué,
- ou alors R₁ et R₂ ou R₂ et R₃ forment ensemble avec le ou les atomes qui les portent, un groupement hétérocycloalkyle éventuellement substitué, ou hétéroaryle éventuellement substitué,
leurs énantiomères, diastéréoisomères, ainsi que leurs sels d'addition à un ou plusieurs acides ou à une ou plusieurs bases pharmaceutiquement acceptables,
étant entendu que :
- le terme alkyle désigne une chaîne hydrocarbonée, linéaire ou ramifiée, contenant de 1 à 6 atomes de carbone,
- le terme alkényle désigne un groupement linéaire ou ramifié contenant de 3 à 6 atomes de carbone et de 1 à 3 doubles liaisons,
- le terme alkynyle désigne un groupement linéaire ou ramifié contenant de 3 à 6 atomes de carbone et de 1 à 3 triples liaisons,
- le terme alkoxy désigne un groupement alkyle-oxy dont la chaîne alkyle, linéaire ou ramifiée, contient de 1 à 6 atomes de carbone,
- le terme aryloxy éventuellement substitué désigne un groupement aryle-oxy dont le groupement aryle est éventuellement substitué,
- le terme acyle désigne un groupement RₐC(O)- où Rₐ représente un atome d'hydrogène ou un groupement alkyle,
- le terme perhalogénoalkyle désigne une chaîne carbonée, linéaire ou ramifiée, contenant de 1 à 3 atomes de carbone et de 1 à 7 atomes d'halogène,
- le terme alkylène désigne un radical bivalent, linéaire ou ramifié, contenant de 1 à 6 atomes de carbone,
- le terme alkénylène désigne un radical bivalent, linéaire ou ramifié, contenant de 2 à 6 atomes de carbone et de 1 à 3 doubles liaisons,
- le terme alkynylène désigne un radical bivalent linéaire ou ramifié, contenant de 2 à 6 atomes de carbones et de 1 à 3 triples liaisons,
- le terme aryle désigne un groupement phényle, naphtyle, indanyle, indényle, dihydronaphtyle ou tétrahydronaphtyle,
- le terme hétéroaryle désigne un groupement monocyclique ou bicyclique dans lequel au moins un des cycles est aromatique, comportant de 5 à 11 chaînons et de 1 à 4 hétéroatomes, choisis parmi l'azote, l'oxygène et le soufre,
- le terme cycloalkyle désigne un monocycle ou bicycle hydrocarboné comportant de 3 à 11 atomes de carbone, et éventuellement insaturé par 1 ou 2 insaturations,
- le terme hétérocycloalkyle désigne un groupement mono ou bicyclique, saturé ou insaturé par 1 ou 2 insaturations, contenant de 4 à 11 chaînons et possédant de 1 à 4 hétéroatomes choisis parmi azote, oxygène et soufre,
- l'expression "éventuellement substitué" affectée aux termes cycloalkyle, aryle, hétéroaryle, hétérocycloalkyle, signifie soit i) que ces groupements peuvent être substitués par 1 à 3 substituants, identiques ou différents, choisis parmi alkyle, alkoxy, alkylthio, alkylsulfinyle, alkylsulfonyle, halogène, hydroxy, mercapto, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo et cyano, soit ii) que ces groupements peuvent être substitués par un groupement aryle, hétéroaryle, cycloalkyle, héterocycloalkyle ou benzyle ; étant entendu que les groupements aryle ou hétéroaryle peuvent être en plus substitués par un ou deux groupements oxo sur la partie non aromatique des groupes ayant une partie aromatique et une partie non aromatique, et les groupements cycloalkyle ou hétérocycloalkyle peuvent être substitués également par un ou deux groupements oxo,
- l'expression « éventuellement substitué » affectée au terme alkyle, alkényle, ou alkynyle, signifie que ces groupements peuvent être substitués par un ou deux groupements, identiques ou différents, choisis parmi alkylthio, alkylsulfinyle, alkylsulfonyle, alkoxy, halogène, hydroxy, mercapto, nitro, amino, acyle, aminocarbonyle, acylamino, alkoxycarbonyle, carboxy, sulfo, cyano, aryle éventuellement substitué, hétéroaryle éventuellement substitué, cycloalkyle éventuellement substitué, hétérocycloalkyle éventuellement substitué, et aryloxy éventuellement substitué.

2. Composés de formule (I) selon la revendication 1 pour lesquels q est égal à 1, leurs énantiomères, diastéréoisomères, ainsi que leurs sels d'addition à un ou plusieurs acides ou une ou plusieurs bases pharmaceutiquement acceptables.

3. Composés de formule (I) selon une quelconque des revendications 1 ou 2 pour lesquels n est égal à 1, leurs énantiomères, diastéréoisomères, ainsi que leurs sels d'addition à un ou plusieurs acides ou une ou plusieurs bases pharmaceutiquement acceptables.

4. Composés de formule (I) selon une quelconque des revendications 1 à 3 pour lesquels m est égal à 1, leurs énantiomères, diastéréoisomères, ainsi que leurs sels d'addition à un ou plusieurs acides ou à une ou plusieurs bases pharmaceutiquement acceptables.

5. Composés de formule (I) selon une quelconque des revendications 1 à 3 pour lesquels m est égal à 2, leurs énantiomères, diastéréoisomères, ainsi que leurs sels d'addition à un ou plusieurs acides ou à une ou plusieurs bases pharmaceutiquement acceptables.

6. Composés de formule (I) selon une quelconque des revendications 1 à 5 pour lesquels p est égal à 1 leurs énantiomères, diastéréoisomères, ainsi que leurs sels d'addition à un ou plusieurs acides ou à une ou plusieurs bases pharmaceutiquement acceptables.

7. Composés de formule (I) selon une quelconque des revendications 1 à 5 pour lesquels p est égal à 2, leurs énantiomères, diastéréoisomères, ainsi que leurs sels d'addition à un ou plusieurs acides ou à une ou plusieurs bases pharmaceutiquement acceptables.

8. Composés de formule (I) selon une quelconque des revendications 1 à 7 pour lesquels X représente un atome d'oxygène ou de soufre, leurs énantiomères, diastéréoisomères, ainsi que leurs sels d'addition à un ou plusieurs acides ou à une ou plusieurs bases pharmaceutiquement acceptables.

9. Composés de formule (I) selon une quelconque des revendications 1 à 7 pour lesquels X représente un groupement -N(R)-, leurs énantiomères, diastéréoisomères, ainsi que leurs sels d'addition à un ou plusieurs acides ou à une ou plusieurs bases pharmaceutiquement acceptables.

10. Composés de formule (I) selon une quelconque des revendications 1 à 9 pour lesquels Y et Y' représentent chacun un atome d'hydrogène, leurs énantiomères, diastéréoisomères, ainsi que leurs sels d'addition à un ou plusieurs acides ou à une ou plusieurs bases pharmaceutiquement acceptables.

11. Composés de formule (I) selon une quelconque des revendications 1 à 9 pour lesquels Y représente un atome d'hydrogène et Y' représente un atome d'halogène ou un groupement alkyle, alkoxy, alkylthio, alkylsulfinyle, allcylsulfonyle, mercapto, hydroxy, perhalogénoalkyle, nitro, amino (non substitué ou substitué par un ou deux groupements alkyle), acyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy, sulfo ou cyano, leurs énantiomères, diastéréoisomères, ainsi que leurs sels d'addition à un ou plusieurs acides ou à une ou plusieurs bases pharmaceutiquement acceptables.

12. Composés de formule (I) selon une quelconque des revendications 1 à 11 pour lesquels Alk représente une chaîne alkylène, leurs énantiomères, diastéréoisomères, ainsi que leurs sels d'addition à un ou plusieurs acides ou à une ou plusieurs bases pharmaceutiquement acceptables.

13. Composés de formule (I) selon une quelconque des revendications 1 à 12 pour lesquels W est situé sur le groupement phényle en position 4, leurs énantiomères, diastéréoisomères, ainsi que leurs sels d'addition à un ou plusieurs acides ou à une ou plusieurs bases pharmaceutiquement acceptables.

14. Composés de formule (I) selon une quelconque des revendications 1 à 13 pour lesquels W représente un groupement cyano, leurs énantiomères, diastéréoisomères, ainsi que leurs sels d'addition à un ou plusieurs acides ou à une ou plusieurs bases pharmaceutiquement acceptables.

15. Composés de formule (I) selon une quelconque des revendications 1 à 13 pour lesquels W représente un groupement N(R₁)-Z₁-R₂, leurs énantiomères, diastéréoisomères, ainsi que leurs sels d'addition à un ou plusieurs acides ou à une ou plusieurs bases pharmaceutiquement acceptables.

16. Composés de formule (I) selon une quelconque des revendications 1 à 13 pour lesquels W représente un groupement -Z₂-NR₁R₂, leurs énantiomères, diastéréoisomères, ainsi que leurs sels d'addition à un ou plusieurs acides ou à une ou plusieurs bases pharmaceutiquement acceptables.

17. Composés de formule (I) selon une quelconque des revendications 1 à 13, ou 16 pour lesquels Z₂ représente un groupement choisi parmi -C(O)-, -C(S) -, -C(NR₄)- et -S(O)ᵣ- leurs énantiomères, diastéréoisomères, ainsi que leurs sels d'addition à un ou plusieurs acides ou à une ou plusieurs bases pharmaceutiquement acceptables.

18. Composés de formule (I) selon une quelconque des revendications 1 à 13, ou 16 pour lesquels Z₂ représente une liaison, leurs énantiomères, diastéréoisomères, ainsi que leurs sels d'addition à un ou plusieurs acides ou à une ou plusieurs bases pharmaceutiquement acceptables.

19. Composés de formule (I) selon une quelconque des revendications 1 à 13, ou 15 pour lesquels Z₁ représente un groupement choisi parmi -C(O)-, -C(S)-, *-C(O)-N(R₃)-, *-C(S)-N(R₃)-, *-C(O)-O- et -S(O)₂-, leurs énantiomères, diastéréoisomères, ainsi que leurs sels d'addition à un ou plusieurs acides ou à une ou plusieurs bases pharmaceutiquement acceptables.

20. Composés de formule (I) selon une quelconque des revendications 1 à 13, ou 15 à 19 pour lesquels R₁, R₂, R₃ et R₄, identiques ou différents, représentent un atome d'hydrogène ou un groupement choisi parmi cycloalkyle ; alkoxy ; phényle éventuellement substitué ; naphtyle ; un groupement hétéroaryle ; et un groupement alkyle éventuellement substitué
- soit par un groupement phényle éventuellement substitué,
- soit par un groupement cycloalkyle,
- soit par un groupement hétérocycloalkyle,
- soit par un groupement hétéroaryle,
- soit par un ou deux groupements alkoxy, ou
- soit par un groupement phényloxy ;
leurs énantiomères, diastéréoisomères, ainsi que leurs sels d'addition à un ou plusieurs acides ou à une ou plusieurs bases pharmaceutiquement acceptables.

21. Composés de formule (I) selon une quelconque des revendications 1 à 13, 15 à 17, ou 19 pour lesquels W représente un groupement choisi parmi N(R₁)-C(O)-NR₂R₃ ; -N(R₁)-C(S)-NR₂R₃ ; -C(O)-NR₁R₂ et -C(S)-NR₁R₂ ; où R₁ et R₂ ou R₂ et R₃ forment ensemble avec le ou les atomes d'azote qui les portent un groupement hétérocycloalkyle ou un groupement pipéridinopipéridinyle, leurs énantiomères, diastéréoisomères, ainsi que leurs sels d'addition à un ou plusieurs acides ou à une ou plusieurs bases pharmaceutiquement acceptables.

22. Composés de formule (I) selon une quelconque des revendications 1 à 13, ou 16 à 18 pour lesquels W représente un groupement -Z₂-NR₁R₂ avec Z₂ représentant une liaison ; R₁, R₂ forment ensemble avec l'atome d'azote qui les porte un groupement hétéroaryle ou alors R₁ représente un atome d'hydrogène ou un groupement alkyle, et R₂ un groupement aryle ou hétéroaryle, leurs énantiomères, diastéréoisomères, ainsi que leurs sels d'addition à un ou plusieurs acides ou à une ou plusieurs bases pharmaceutiquement acceptables.

23. Composés de formule (I) selon une quelconque des revendications 1 à 13, 16, 17 ou 21 pour lesquels W représente un groupement -C(O)-NR₁R₂ où R₁, R₂ forment ensemble avec l'atome d'azote qui les porte un groupement choisi parmi pipérazinyle éventuellement substitué par un groupement alkyle ou benzyle ; pipéridinyle éventuellement substitué par un groupement alkyle ou benzyle ; morpholinyle ; azépanyle ; thiomorpholinyle ; octahydrocyclopentapyrrolyle ; dihydroquinolinyle ; et tétrahydro-quinolinyle, leurs énantiomères, diastéréoisomères, ainsi que leurs sels d'addition à un ou plusieurs acides ou à une ou plusieurs bases pharmaceutiquement acceptables.

24. Composés de formule (I) selon une quelconque des revendications 1 à 13, 16, 17 ou 20 pour lesquels W représente un groupement -C(O)NR₁R₂ où R₁, R₂ représentent indépendamment un groupement alkyle ou un atome d'hydrogène, leurs énantiomères, diastéréoisomères, ainsi que leurs sels d'addition à un ou plusieurs acides ou à une ou plusieurs bases pharmaceutiquement acceptables.

25. Composés de formule (I) selon une quelconque des revendications 1 à 13, 15, 19 ou 20 pour lesquels W représente un groupement -N(R₁) -C(O)-R₂ où R₁, R₂ représentent indépendamment un groupement alkyle ou un atome d'hydrogène, leurs énantiomères, diastéréoisomères, ainsi que leurs sels d'addition à un ou plusieurs acides ou à une ou plusieurs bases pharmaceutiquement acceptables.

26. Composé de formule (I) selon une quelconque des revendications 1 à 14 qui est le 4-(3-hexahydrocyclopenta[*c*]pyrrol-2(1*H*)ylpropoxy)benzonitrile, ses énantiomères, diastéréoisomères, ainsi que ses sels d'addition à un acide pharmaceutiquement acceptable.

27. Composé de formule (I) selon une quelconque des revendications 1 à 13, 16, 17, 20 ou 24 qui est le 4-(3-hexahydrocyclopenta[*c*]-pyrrol-2(1*H*)-ylpropoxy)benzamide, ses énantiomères, diastéréoisomères, ainsi que ses sels d'addition à acide pharmaceutiquement acceptable.

28. Composé de formule (I) selon une quelconque des revendications 1 à 13, 16, 17, 20 ou 24 qui est le 4-[3-(hexahydrocyclopenta[c]pyrrol-2(1H)-yl)propoxy]-N-méthyl-benzamide, ses énantiomères, diastéréoisomères, ainsi que ses sels d'addition à un acide pharmaceutiquement acceptable.

29. Composé de formule (I) selon une quelconque des revendications 1 à 13, 16, 17, 20 ou 24 qui est le 4-[3-(hexahydrocyclopenta[*c*]pyrrol-2(1*H*)-yl)propoxy]-*N,N*-diméthylbenzamide, ses énantiomères, diastéréoisomères, ainsi que ses sels d'addition à un acide pharmaceutiquement acceptable.

30. Composé de formule (I) selon une quelconque des revendications 1 à 13, 15, 19, 20 ou 25 qui est le *N*-[4-(3-hexahydrocyclopenta[*c*]pyrrol-2(1*H*)-ylpropoxy)phényl]acétamide, ses énantiomères, diastéréoisomères, ainsi que ses sels d'addition à un acide pharmaceutiquement acceptable.

31. Procédé de préparation des composés de formule (I) selon la revendication 1 **caractérisé en ce que** l'on utilise comme produit de départ un composé de formule (II) : 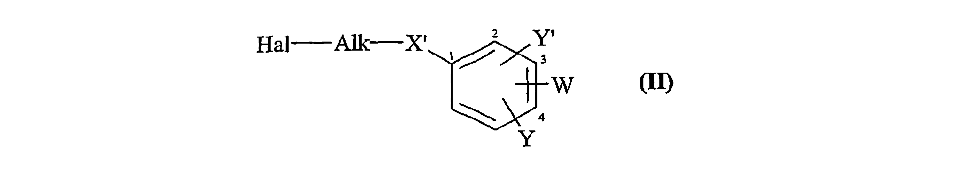 dans laquelle :
Alk est tel que défini dans la formule (I), Hal représente un atome d'halogène, X' représente un atome d'oxygène, de soufre ou un groupement -N(p)-, où (p) représente un atome d'hydrogène, un groupement de protection classique de l'atome d'azote ou un groupement alkyle, W, Y et Y' sont tels que définis dans la formule (I),
composé de formule (II) qui après déprotection éventuelle, se condense en milieu basique avec un bicycle de formule (III) : 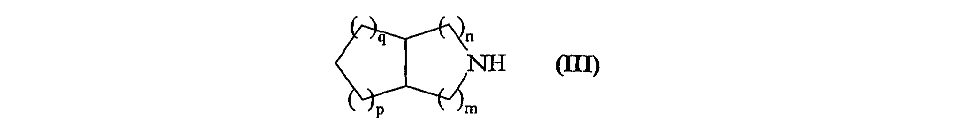 dans laquelle :
n, m, p et q sont tels que définis dans la formule (I), pour conduire au composé de formule (I),
■ composé de formule (I) qui, lorsque W représente un groupement cyano, réagit éventuellement avec la soude ou la potasse,
pour conduire au composé de formule (I/b) : 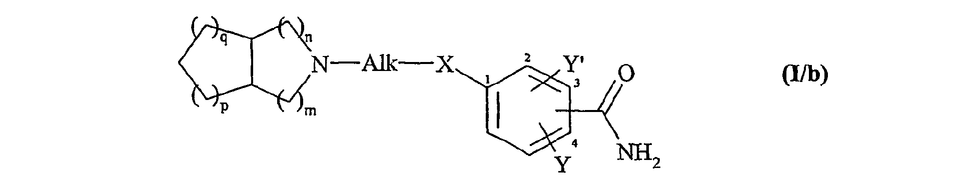 cas particulier des composés de formule (I) pour lequel Alk, n, m, p, q, X, Y et Y' sont tels que définis dans la formule (I),
composés de formule (I),
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un ou plusieurs acides ou à une ou plusieurs bases pharmaceutiquement acceptables,
étant entendu :
- qu'à tout moment jugé opportun au cours du procédé précédemment décrit, le ou les groupements carbonyle, thiocarbonyle, amino, alkylamino du réactif de départ (II) peuvent être protégés puis, après condensation, déprotégés pour les besoins de la synthèse,
- que les réactifs (II), et (III) sont préparés selon des modes opératoires connus, décrits dans la littérature.

32. Compositions pharmaceutiques contenant comme principe actif au moins un composé selon l'une quelconque des revendications 1 à 30, seul ou en combinaison avec un ou plusieurs excipients ou véhicules inertes, non toxiques, pharmaceutiquement acceptables.

33. Compositions pharmaceutiques selon la revendication 32 contenant au moins un principe actif selon l'une quelconque des revendications 1 à 30 utiles, en tant que médicament, dans le traitement des déficits cognitifs associés au vieillissement cérébral et aux maladies neurodégénératives, ainsi que dans le traitement des troubles de l'humeur, des crises convulsives, du syndrome d'hyperactivité avec déficits attentionnels, de l'obésité et de la douleur.

34. Compositions pharmaceutiques selon la revendication 32 contenant au moins un principe actif selon l'une quelconque des revendications 1 à 30 utiles, en tant que médicament, dans le traitement des déficits cognitifs associés à la maladie d'Alzheimer, la maladie de Parkinson, la maladie de Pick, la maladie de Korsakoff, et les démences frontales et sous-corticales d'origines vasculaires ou autres.

35. Utilisation de composition pharmaceutique selon la revendication 32 contenant au moins un principe actif selon l'une quelconque des revendications 1 à 30 pour la fabrication de médicaments utiles dans le traitement des déficits cognitifs associés au vieillissement cérébral et aux maladies neurodégénératives, ainsi que dans le traitement des troubles de l'humeur, des crises convulsives, du syndrome d'hyperactivité avec déficits attentionnels, de l'obésité et de la douleur.

36. Utilisation de la composition pharmaceutique selon la revendication 32 contenant au moins un principe actif selon l'une quelconque des revendications 1 à 30 pour la fabrication de médicaments utiles dans le traitement des déficits cognitifs associés à la maladie d'Alzheimer, la maladie de Parkinson, la maladie de Pick, la maladie de Korsakoff, et les démences frontales et sous-corticales d'origines vasculaires ou autres

## Claims

1. Compounds of formula (I) : 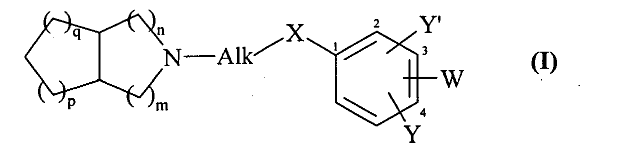 wherein:
• **m** and **n**, which may be identical or different, each represent an integer of from 0 to 2 inclusive, with the sum of the two integers being from 2 to 3 inclusive,
• **p** and **q**, which may be identical or different, each represent an integer of from 0 to 2 inclusive,
• **Alk** represents an alkylene, alkenylene or alkynylene chain,
• **Y** and **Y',** which may be identical or different, each represent a hydrogen atom, a halogen atom or an alkyl, alkoxy, alkylthio, alkylsulphinyl, alkylsulphonyl, mercapto, hydroxy, perhaloalkyl, nitro, amino (unsubstituted or substituted by one or two alkyl groups), acyl, aminocarbonyl (optionally substituted on the nitrogen atom by one or two alkyl groups), acylamino (optionally substituted on the nitrogen atom by an alkyl group), alkoxycarbonyl, carboxy, sulpho or cyano group,
• **X** represents an oxygen atom, a sulphur atom or an -N(R)- group wherein R represents a hydrogen atom or an alkyl group,
• **W** represents a group selected from cyano (when X represents an oxygen atom or an -N(R)- group), -N(R₁)-Z₁-R₂ and -Z₂-NR₁R₂,
wherein :
- Z₁ represents -C(O)-, -C(S)-, -C(NR₄)-, *-C(O)-N(R₃)-, *-C(S)-N(R₃)-, *-C(NR₄)-N(R₃)-, *-C(O)-O-, *-C(S)-O- or -S(O)ᵣ-, in which r = 1 or 2, and * corresponds to the point of attachment to N(R₁),
- Z₂ represents -C(O)-, -C(S)-, -C(NR₄)-, -S(O)ᵣ- or a bond,
- R₁, R₂, R₃ and R₄, which may be identical or different, each represent a hydrogen atom, an optionally substituted alkyl group, optionally substituted alkenyl group, optionally substituted alkynyl group, alkoxy group, optionally substituted cycloalkyl group, optionally substituted heterocycloalkyl group, optionally substituted aryl group or optionally substituted heteroaryl group,
- or R₁ and R₂ or R₂ and R₃, together with the atom or atoms carrying them, form an optionally substituted heterocycloalkyl or optionally substituted heteroaryl group,
their enantiomers, diastereoisomers, and also addition salts thereof with one or more pharmaceutically acceptable acids or with one or more pharmaceutically acceptable bases,
wherein:
- the term alkyl denotes a linear or branched hydrocarbon chain containing from 1 to 6 carbon atoms,
- the term alkenyl denotes a linear or branched group containing from 3 to 6 carbon atoms and from 1 to 3 double bonds,
- the term alkynyl denotes a linear or branched group containing from 3 to 6 carbon atoms and from 1 to 3 triple bonds,
- the term alkoxy denotes an alkyl-oxy group in which the linear or branched alkyl chain contains from 1 to 6 carbon atoms,
- the expression optionally substituted aryloxy denotes an aryl-oxy group of which the aryl group is optionally substituted,
- the term acyl denotes an RₐC(O)- group in which Rₐ represents a hydrogen atom or an alkyl group,
- the term perhaloalkyl denotes a linear or branched carbon chain containing from 1 to 3 carbon atoms and from 1 to 7 halogen atoms,
- the term alkylene denotes a linear or branched bivalent radical containing from 1 to 6 carbon atoms,
- the term alkenylene denotes a linear or branched bivalent radical containing from 2 to 6 carbon atoms and from 1 to 3 double bonds,
- the term alkynylene denotes a linear or branched bivalent radical containing from 2 to 6 carbon atoms and from 1 to 3 triple bonds,
- the term aryl denotes a phenyl, naphthyl, indanyl, indenyl, dihydronaphthyl or tetrahydronaphthyl group,
- the term heteroaryl denotes a monocyclic or bicyclic group in which at least one of the rings is aromatic, the group containing from 5 to 11 ring members and from 1 to 4 hetero atoms selected from nitrogen, oxygen and sulphur,
- the term cycloalkyl denotes a hydrocarbon monocycle or bicycle containing from 3 to 11 carbon atoms and optionally unsaturated by 1 or 2 unsaturated bonds,
- the term heterocycloalkyl denotes a mono- or bi-cyclic group, saturated or unsaturated by 1 or 2 unsaturated bonds, containing from 4 to 11 ring members and having from 1 to 4 hetero atoms selected from nitrogen, oxygen and sulphur,
- the expression "optionally substituted" applied to the terms cycloalkyl, aryl, heteroaryl and heterocycloalkyl denotes either i) that those groups may be substituted by from 1 to 3 identical or different substituents selected from alkyl, alkoxy, alkylthio, alkylsulphinyl, alkylsulphonyl, halogen, hydroxy, mercapto, perhaloalkyl, nitro, amino (unsubstituted or substituted by one or two alkyl groups), acyl, aminocarbonyl (optionally substituted on the nitrogen atom by one or two alkyl groups), acylamino (optionally substituted on the nitrogen atom by an alkyl group), alkoxycarbonyl, carboxy, sulpho and cyano, or ii) that those groups may be substituted by an aryl, heteroaryl, cycloalkyl, heterocycloalkyl or benzyl group; it being understood that the aryl or heteroaryl groups may in addition be substituted by one or two oxo groups on the non-aromatic moiety of the groups having an aromatic moiety and a non-aromatic moiety, and that the cycloalkyl or heterocycloalkyl groups may likewise be substituted by one or two oxo groups,
- the expression "optionally substituted" applied to the term alkyl, alkenyl or alkynyl denotes that those groups may be substituted by one or two identical or different groups selected from alkylthio, alkylsulphinyl, alkylsulphonyl, alkoxy, halogen, hydroxy, mercapto, nitro, amino, acyl, aminocarbonyl, acylamino, alkoxycarbonyl, carboxy, sulpho, cyano, optionally substituted aryl, optionally substituted heteroaryl, optionally substituted cycloalkyl, optionally substituted heterocycloalkyl and optionally substituted aryloxy.

2. Compounds of formula (I) according to claim 1, wherein q is 1, their enantiomers, diastereoisomers, and also addition salts thereof with one or more pharmaceutically acceptable acids or one or more pharmaceutically acceptable bases.

3. Compounds of formula (I) according to claim 1 or 2, wherein n is 1, their enantiomers, diastereoisomers, and also addition salts thereof with one or more pharmaceutically acceptable acids or one or more pharmaceutically acceptable bases.

4. Compounds of formula (I) according to any one of claims 1 to 3, wherein m is 1, their enantiomers, diastereoisomers, and also addition salts thereof with one or more pharmaceutically acceptable acids or with one or more pharmaceutically acceptable bases.

5. Compounds of formula (I) according to any one of claims 1 to 3, wherein m is 2, their enantiomers, diastereoisomers, and also addition salts thereof with one or more pharmaceutically acceptable acids or with one or more pharmaceutically acceptable bases.

6. Compounds of formula (I) according to any one of claims 1 to 5, wherein p is 1, their enantiomers, diastereoisomers, and also addition salts thereof with one or more pharmaceutically acceptable acids or with one or more pharmaceutically acceptable bases.

7. Compounds of formula (I) according to any one of claims 1 to 5, wherein p is 2, their enantiomers, diastereoisomers, and also addition salts thereof with one or more pharmaceutically acceptable acids or with one or more pharmaceutically acceptable bases.

8. Compounds of formula (I) according to any one of claims 1 to 7, wherein X represents an oxygen atom or a sulphur atom, their enantiomers, diastereoisomers, and also addition salts thereof with one or more pharmaceutically acceptable acids or with one or more pharmaceutically acceptable bases.

9. Compounds of formula (I) according to any one of claims 1 to 7, wherein X represents an -N(R)- group, their enantiomers, diastereoisomers, and also addition salts thereof with one or more pharmaceutically acceptable acids or with one or more pharmaceutically acceptable bases.

10. Compounds of formula (I) according to any one of claims 1 to 9, wherein Y and Y' each represent a hydrogen atom, their enantiomers, diastereoisomers, and also addition salts thereof with one or more pharmaceutically acceptable acids or with one or more pharmaceutically acceptable bases.

11. Compounds of formula (I) according to any one of claims 1 to 9, wherein Y represents a hydrogen atom and Y' represents a halogen atom or an alkyl, alkoxy, alkylthio, alkylsulphinyl, alkylsulphonyl, mercapto, hydroxy, perhaloalkyl, nitro, amino (unsubstituted or substituted by one or two alkyl groups), acyl, aminocarbonyl (optionally substituted on the nitrogen atom by one or two alkyl groups), acylamino (optionally substituted on the nitrogen atom by an alkyl group), alkoxycarbonyl, carboxy, sulpho or cyano group, their enantiomers, diastereoisomers, and also addition salts thereof with one or more pharmaceutically acceptable acids or with one or more pharmaceutically acceptable bases.

12. Compounds of formula (I) according to any one of claims 1 to 11, wherein Alk represents an alkylene chain, their enantiomers, diastereoisomers, and also addition salts thereof with one or more pharmaceutically acceptable acids or with one or more pharmaceutically acceptable bases.

13. Compounds of formula (I) according to any one of claims 1 to 12, wherein W is located on the phenyl group in the 4-position, their enantiomers, diastereoisomers, and also addition salts thereof with one or more pharmaceutically acceptable acids or with one or more pharmaceutically acceptable bases.

14. Compounds of formula (I) according to any one of claims 1 to 13, wherein W represents a cyano group, their enantiomers, diastereoisomers, and also addition salts thereof with one or more pharmaceutically acceptable acids or with one or more pharmaceutically acceptable bases.

15. Compounds of formula (I) according to any one of claims 1 to 13, wherein W represents an -N(R₁)-Z₁-R₂ group, their enantiomers, diastereoisomers, and also addition salts thereof with one or more pharmaceutically acceptable acids or with one or more pharmaceutically acceptable bases.

16. Compounds of formula (I) according to any one of claims 1 to 13, wherein W represents a -Z₂-NR₁R₂ group, their enantiomers, diastereoisomers, and also addition salts thereof with one or more pharmaceutically acceptable acids or with one or more pharmaceutically acceptable bases.

17. Compounds of formula (I) according to any one of claims 1 to 13 and 16, wherein Z₂ represents a group selected from -C(O)-, -C(S)-, -C(NR₄)- and -S(O)ᵣ-, their enantiomers, diastereoisomers, and also addition salts thereof with one or more pharmaceutically acceptable acids or with one or more pharmaceutically acceptable bases.

18. Compounds of formula (I) according to any one of claims 1 to 13 and 16, wherein Z₂ represents a bond, their enantiomers, diastereoisomers, and also addition salts thereof with one or more pharmaceutically acceptable acids or with one or more pharmaceutically acceptable bases.

19. Compounds of formula (I) according to any one of claims 1 to 13 and 15, wherein Z₁ represents a group selected from -C(O)-, -C(S)-, *-C(O)-N(R₃)-, *-C(S)-N(R₃)-, *-C(O)-O- and -S(O)₂-, their enantiomers, diastereoisomers, and also addition salts thereof with one or more pharmaceutically acceptable acids or with one or more pharmaceutically acceptable bases.

20. Compounds of formula (I) according to any one of claims 1 to 13 and 15 to 19, wherein R₁, R₂, R₃ and R₄, which may be identical or different, each represent a hydrogen atom or a group selected from cycloalkyl; alkoxy; optionally substituted phenyl; naphthyl; a heteroaryl group; and an alkyl group optionally substituted
- by an optionally substituted phenyl group,
- or by a cycloalkyl group,
- or by a heterocycloalkyl group,
- or by a heteroaryl group,
- or by one or two alkoxy groups, or
- by a phenyloxy group;
their enantiomers, diastereoisomers, and also addition salts thereof with one or more pharmaceutically acceptable acids or with one or more pharmaceutically acceptable bases.

21. Compounds of formula (I) according to any one of claims 1 to 13, 15 to 17 and 19, wherein W represents a group selected from N(R₁)-C(O)-NR₂R₃; -N(R₁)-C(S)-NR₂R₃; -C(O)-NR₁R₂ and -C(S)-NR₁R₂; wherein R₁ and R₂ or R₂ and R₃, together with the nitrogen atom or atoms carrying them, form a heterocycloalkyl group or a piperidino-piperidyl group, their enantiomers, diastereoisomers, and also addition salts thereof with one or more pharmaceutically acceptable acids or with one or more pharmaceutically acceptable bases.

22. Compounds of formula (I) according to any one of claims 1 to 13 and 16 to 18, wherein W represents a -Z₂-NR₁R₂ group in which Z₂ represents a bond ;
R₁ and R₂, together with the nitrogen atom carrying them, form a heteroaryl group or R₁ represents a hydrogen atom or an alkyl group and R₂ represents an aryl or heteroaryl group, their enantiomers, diastereoisomers, and also addition salts thereof with one or more pharmaceutically acceptable acids or with one or more pharmaceutically acceptable bases.

23. Compounds of formula (I) according to any one of claims 1 to 13, 16, 17 and 21, wherein W represents a -C(O)-NR₁R₂ group in which R₁ and R₂, together with the nitrogen atom carrying them, form a group selected from piperazinyl optionally substituted by an alkyl or benzyl group; piperidyl optionally substituted by an alkyl or benzyl group; morpholinyl; azepanyl; thiomorpholinyl; octahydrocyclopentapyrrolyl; dihydroquinolinyl; and tetrahydroquinolinyl, their enantiomers, diastereoisomers, and also addition salts thereof with one or more pharmaceutically acceptable acids or with one or more pharmaceutically acceptable bases.

24. Compounds of formula (I) according to any one of claims 1 to 13, 16, 17 and 20, wherein W represents a -C(O)-NR₁R₂ group in which R₁ and R₂, independently, each represent an alkyl group or a hydrogen atom, their enantiomers, diastereoisomers, and also addition salts thereof with one or more pharmaceutically acceptable acids or with one or more pharmaceutically acceptable bases.

25. Compounds of formula (I) according to any one of claims 1 to 13, 15, 19 and 20, wherein W represents a -N(R₁)-C(O)-R₂ group in which R₁ and R₂, independently, each represent an alkyl group or a hydrogen atom, their enantiomers, diastereoisomers, and also addition salts thereof with one or more pharmaceutically acceptable acids or with one or more pharmaceutically acceptable bases.

26. Compound of formula (I) according to any one of claims 1 to 14, which is 4-(3-hexahydrocyclopenta[c]pyrrol-2(1H)-ylpropoxy)benzonitrile, its enantiomers, diastereoisomers, and also addition salts thereof with a pharmaceutically acceptable acid.

27. Compound of formula (I) according to any one of claims 1 to 13, 16, 17, 20 and 24, which is 4-(3-hexahydrocyclopenta[*c*]-pyrrol-2(1*H*)-ylpropoxy)benzamide, its enantiomers, diastereoisomers, and also addition salts thereof with a pharmaceutically acceptable acid.

28. Compound of formula (I) according to any one of claims 1 to 13, 16, 17, 20 and 24, which is 4-[3-(hexahydrocyclopenta[*c*]pyrrol-2(1*H*)-yl)propoxy]-*N-*methyl-benzamide, its enantiomers, diastereoisomers, and also addition salts thereof with a pharmaceutically acceptable acid.

29. Compound of formula (I) according to any one of claims 1 to 13, 16, 17, 20 and 24, which is 4-[3-(hexahydrocyclopenta[*c*]pyrrol-2(1H-yl)propoxy]-*N*,*N*-dimethyl-benzamide, its enantiomers, diastereoisomers, and also addition salts thereof with a pharmaceutically acceptable acid.

30. Compound of formula (I) according to any one of claims 1 to 13, 15, 19, 20 and 25, which is *N*-[4-(3-hexahydrocyclopenta[*c*]pyrrol-2(1*H*)-ylpropoxy)phenyl]acetamide, its enantiomers, diastereoisomers, and also addition salts thereof with a pharmaceutically acceptable acid.

31. Process for the preparation of the compounds of formula (I) according to claim 1, **characterised in that** there is used as starting material a compound of formula (II) : 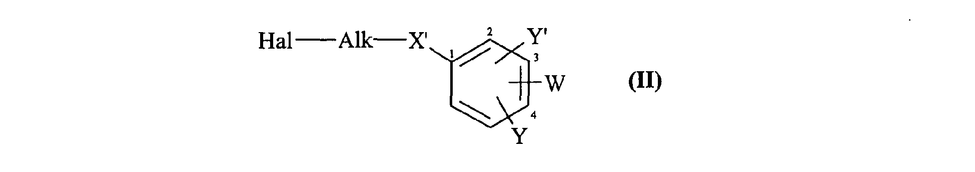 wherein :
Alk is as defined for formula (I), Hal represents a halogen atom, X' represents an oxygen atom, a sulphur atom or an -N(p)- group, in which (p) represents a hydrogen atom, a conventional protecting group for the nitrogen atom, or an alkyl group, and
W, Y and Y' are as defined for formula (I),
which compound of formula (II), after optional deprotection, is condensed in basic medium with a bicycle of formula (III) : 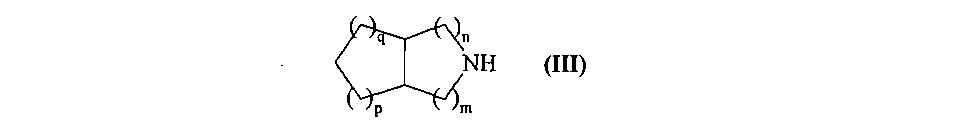 wherein :
n, m, p and q are as defined for formula (I), to yield a compound of formula (I),
■ which compound of formula (I), when W represents a cyano group, is optionally reacted with sodium hydroxide or potassium hydroxide
to yield a compound of formula (I/b) : 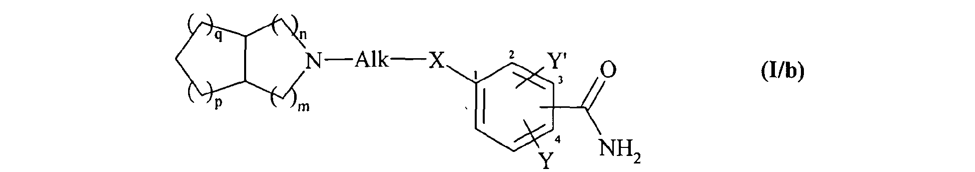 a particular case of the compounds of formula (I) wherein Alk, n, m, p, q, X, Y and Y' are as defined for formula (I),
which compounds of formula (I),
- may, if desired, be purified according to a conventional purification technique,
- are optionally separated into stereoisomers according to a conventional separation technique,
- are converted, if desired, into addition salts with one or more pharmaceutically acceptable acids or with one or more pharmaceutically acceptable bases,
it being understood that:
- at any moment considered appropriate during the course of the process described above, the group or groups carbonyl, thiocarbonyl, amino, alkylamino of the starting reagent (II) can be protected and then, after condensation, deprotected, as required by the synthesis,
- the reagents (II) and (III) are prepared according to known procedures described in the literature.

32. Pharmaceutical compositions containing as active ingredient at least one compound according to any one of claims 1 to 30, alone or in combination with one or more pharmaceutically acceptable, inert, non-toxic excipients or carriers.

33. Pharmaceutical compositions according to claim 32, containing at least one active ingredient according to any one of claims 1 to 30, for use as a medicament in the treatment of cognitive deficiencies associated with cerebral ageing and with neurodegenerative diseases, and also in the treatment of mood disorders, convulsive attacks, attention deficit hyperactivity syndrome, obesity and pain.

34. Pharmaceutical compositions according to claim 32, containing at least one active , ingredient according to any one of claims 1 to 30, for use as a medicament in the treatment of cognitive deficiencies associated with Alzheimer's disease, Parkinson's disease, Pick's disease, Korsakoff disease, and frontal and sub-cortical dementias of vascular or other origins.

35. Use of a pharmaceutical composition according to claim 32, containing at least one active ingredient according to any one of claims 1 to 30, for the manufacture of medicaments for use in the treatment of cognitive deficiencies associated with cerebral ageing and with neurodegenerative diseases, and also in the treatment of mood disorders, convulsive attacks, attention deficit hyperactivity syndrome, obesity and pain.

36. Use of the pharmaceutical composition according to claim 32, containing at least one active ingredient according to any one of claims 1 to 30, for the manufacture of medicaments for use in the treatment of cognitive deficiencies associated with Alzheimer's disease, Parkinson's disease, Pick's disease, Korsakoffs disease, and frontal and sub-cortical dementias of vascular or other origins.

## Patentansprüche

1. Verbindungen der Formel (I): 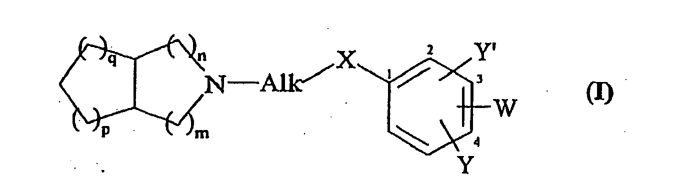 in der:
• m und n, die gleich oder verschieden sind, eine ganze Zahl zwischen 0 und 2 einschließlich bedeuten, wobei die Summe der beiden ganzen Zahlen 2 und 3 einschließlich beträgt,
• p und q, die gleich oder verschieden sind, eine ganze Zahl zwischen 0 und 2 einschließlich bedeuten,
• Alk eine Alkylen-, Alkenylen- oder Alkinylen-kette bedeutet,
• Y und Y', die gleich oder verschieden sind, ein Wasserstoffatom, Halogenatom oder eine Alkyl-, Alkoxy-, Alkylthio-, Alkylsulfinyl-, Alkylsulfonyl-, Mercapto-, Hydroxy-, Perhalogenakyl-, Nitro-, Amino- (die nichtsubstituiert oder durch eine oder zwei Alkylgruppen substituiert ist), Acyl-, Aminocarbonyl- (die gegebenenfalls am Stickstoffatom durch eine oder zwei Alkylgruppen substituiert ist), Acylamino- (die gegebenenfalls am Stickstoffatom durch eine Alkylgruppe substituiert ist), Alkoxycarbonyl-, Carboxy-, Sulfo- oder Cyanogruppe bedeuten,
• X ein Sauerstoffatom, Schwefelatom oder eine Gruppe -N(R)- bedeutet, worin R ein Wasserstoffatom oder eine Alkylgruppe darstellt,
• W eine Gruppe bedeutet ausgewählt aus Cyano (wenn X ein Sauerstoffatom oder eine Gruppe N(R) darstellt), -N-(R₁)-Z₁-R₂ und -Z₂-NR₁R₂,
worin:
- Z₁ -C(O)-, -C(S)-, -C(NR₄)-, *-C(O)-N(R₃)-, *-C(S)-N(R₃)-, *-C(NR₄)-N(R₃)-, *-C(O)-O-, *-C(S)-O-, -S(O)ᵣ- darstellt, worin r = 1 oder 2 und * den Bindungspunkt an N(R₁) darstellt,
- Z₂ -C(O)-, -C(S)-, -C(NR₄)-, -S(O)ᵣ- oder eine Bindung darstellt,
- R₁, R₂, R₃ und R₄, die gleich oder verschieden sind, ein Wasserstoffatom, eine gegebenenfalls substituierte Alkyl-, gegebenenfalls substituierte Alkenyl-, gegebenenfalls substituierte Alkinyl-, Alkoxy-, gegebenenfalls substituierte Cycloalkyl-, gegebenenfalls substituierte Heterocycloalkyl-, gegebenenfalls substituierte Aryl- oder gegebenenfalls substituierte Heteroarylgruppe darstellen,
- oder R₁ und R₂ oder R₂ und R₃ gemeinsam mit dem oder den sie tragenden Atomen eine gegebenenfalls substituierte Heterocycloalkyl- oder gegebenenfalls substituierte Heteroarylgruppe bilden,
deren Enantiomere, Diastereoisomere sowie deren Additionssalze mit einer oder mehreren pharmazeutisch annehmbaren Säuren oder einer oder mehreren pharmazeutisch annehmbaren Basen,
mit der Maßgabe, daß:
- der Begriff Alkyl für eine geradkettige oder verzweigte Kohlenwasserstoffkette steht, die 1 bis 6 Kohlenstoffatome enthält,
- der Begriff Alkenyl für eine geradkettige oder verzweigte Gruppe steht, die 3 bis 6 Kohlenstoffatome und 1 bis 3 Doppelbindungen enthält,
- der Begriff Alkinyl für eine geradkettige oder verzweigte Gruppe steht, die 3 bis 6 Kohlenstoffatome und 1 bis 3 Dreifachbindungen enthält,
- der Begriff Alkoxy für eine Alkyl-oxygruppe steht, deren geradkettige oder verzweigte Alkylkette 1 bis 6 Kohlenstoffatome enthält,
- der Begriff gegebenenfalls substituiertes Aryloxy für eine Aryl-oxygruppe steht, deren Arylgruppe gegebenenfalls substituiert ist,
- der Begriff Acyl für eine Gruppe RₐC(O)- steht, worin Rₐ ein Wasserstoffatom oder eine Alkylgruppe bedeutet,
- der Begriff Perhalogenalkyl für eine geradkettige oder verzweigte kohlenstoffhaltige Kette steht, die 1 bis 3 Kohlenstoffatome und 1 bis 7 Halogenatome enthält,
- der Begriff Alkylen für einen geradkettigen oder verzweigten zweiwertigen Rest steht, der 1 bis 6 Kohlenstoffatome enthält,
- der Begriff Alkenylen für einen geradkettigen oder verzweigten zweiwertigen Rest steht, der 2 bis 6 Kohlenstoffatome und 1 bis 3 Doppelbindungen enthält,
- der Begriff Alkinylen für einen geradkettigen oder verzweigten zweiwertigen Rest steht, der 2 bis 6 Kohlenstoffatome und- 1 bis- 3 Dreifachbindungen enthält.
- der Begriff Aryl für eine Phenyl-, Naphthyl-, Indanyl-, Indenyl-, Dihydronaphthyl- oder Tetrahydronaphthylgruppe steht,
- der Begriff Heteroaryl für eine monocyclische oder bicyclische Gruppe steht, in der mindestens einer der Ringe aromatisch ist und die 5 bis 11 Kettenglieder und 1 bis 4 Heteroatome ausgewählt aus Stickstoff, Sauerstoff und Schwefel enthält,
- der Begriff Cycloalkyl für einen monocyclischen oder bicyclischen Kohlenwasserstoffrest steht, der 3 bis 11 Kohlenstoffatome aufweist und gegebenenfalls durch eine oder zwei Unsättigungen ungesättigt ist,
- der Begriff Heterocycloalkyl eine gesättigte oder durch 1 oder 2 Unsättigungen ungesättigte mono- oder bicyclische Gruppe steht, die 4 bis 11 Kettenglieder enthält und 1 bis 4 Heteroatome ausgewählt aus Stickstoff, Sauerstoff und Schwefel aufweist,
- der Begriff "gegebenenfalls substituiert" im Hinblick auf die Begriffe Cycloalkyl, Aryl, Heteroaryl und Heterocycloalkyl entweder bedeutet, i) daß diese Gruppen durch 1 bis 3 gleiche oder verschiedene Substituenten ausgewählt aus Alkyl, Alkoxy, Alkythio, Alkylsulfinyl, Alkylsulfonyl, Halogen, Hydroxy, Mercapto, Perhalogenoalkyl, Nitro, Amino (nichtsubsituiert oder durch eine oder zwei Alkylgruppen substituiert), Acyl, Aminocarbonyl (gegebenenfalls am Stickstoffatom durch eine oder zwei Alkylgruppen substituiert), Acylamino (gegebenenfalls am Stickstoffatom durch eine Alkylgruppe substituiert), Alkoxycarbonyl, Carboxy, Sulfo und Cyano substituiert sein können oder ii) daß diese Gruppen durch eine Aryl-, Heteroaryl-, Cycloalkyl-, Heterocycloalkyl- oder Benzylgruppe substituiert sein können; mit der Maßgabe, daß die Aryl- oder Heteroarylgruppen zusätzlich durch eine oder zwei Oxogruppen am nichtaromatischen Rest von Gruppen substituiert sein können, die einen aromatischen und einen nichtaromatischen Teil aufweisen, und die Cycloalkyl- oder Heterocycloalkylgruppen ihrerseits durch eine oder zwei Oxogruppen substituiert sein können,
- der Begriff " gegebenenfalls substituiert" im Hinblick auf die Begriffe Alkyl, Alkenyl oder Alkinyl bedeutet, daß diese Gruppen durch eine oder zwei gleiche oder verschiedene Gruppen ausgewählt aus Alkylthio, Alkylsulfinyl, Alkylsulfonyl, Alkoxy, Halogen, Hydroxy, Mercapto, Nitro, Amino, Acyl, Aminocarbonyl, Acylamino, Alkoxycarbonyl, Carboxy, Sulfo, Cyano, gegebenenfalls substituiertes Aryl, gegebenenfalls substituiertes Heteroaryl, gegebenenfalls substituiertes Cycloalkyl, gegebenenfalls substituiertes Heterocycloalkyl und gegebenenfalls substituiertes Aryloxy substituiert sein können.

2. Verbindungen der Formel (I) nach Anspruch 1, worin q den Wert 1 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

3. Verbindungen der Formel (I) nach einem der Ansprüche 1 oder 2, worin n den Wert 1 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

4. Verbindungen der Formel (I) nach einem der Ansprüche 1 bis 3, worin m den Wert 1 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

5. Verbindungen der Formel (I) nach einem der Ansprüche 1 bis 3, worin m den Wert 2 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

6. Verbindungen der Formel (I) nach einem der Ansprüche 1 bis 5, worin p den Wert 1 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

7. Verbindungen der Formel (I) nach einem der Ansprüche 1 bis 5, worin p den Wert 2 besitzt, deren Enantiomere, Diastereoisomere sowie deren Additionssalze mit einer oder mehreren pharmazeutisch annehmbaren Säuren oder einer oder mehreren pharmazeutisch annehmbaren Basen.

8. Verbindungen der Formel (I) nach einem der Ansprüche 1 bis 7, worin X ein Sauerstoffatom oder ein Schwefelatom bedeutet, deren Enantiomere, Diastereoisomere sowie deren Additionssalze mit einer oder mehreren pharmazeutisch annehmbaren Säuren oder einer oder mehreren pharmazeutisch annehmbaren Basen.

9. Verbindungen der Formel (I) nach einem der Ansprüche 1 bis 7, worin X eine Gruppe -N(R)- bedeutet, deren Enantiomere, Diastereoisomere sowie deren Additionssalze mit einer oder mehreren pharmazeutisch annehmbaren Säuren oder einer oder mehreren pharmazeutisch annehmbaren Basen.

10. Verbindungen der Formel (I) nach einem der Ansprüche 1 bis 9, worin Y und Y' jeweils ein Wasserstoffatom bedeuten, deren Enantiomere, Diastereoisomere sowie deren Additionssalze mit einer oder mehreren pharmazeutisch annehmbaren Säuren oder einer oder mehreren pharmazeutisch annehmbaren Basen.

11. Verbindungen der Formel (I) nach einem der Ansprüche 1 bis 9, worn Y ein Wasserstoffatom und Y' ein Halogenatom oder eine Alkyl-, Alkoxy-, Alkylthio-, Alkylsulfinyl-, Alkylsulfonyl-, Mercapto-, Hydroxy-, Perhalogenalkyl-, Nitro-, Amino-(nichtsubstituiert oder durch eine oder zwei Alkylgruppen substituiert), Acyl-, Aminocarbonyl- (gegebenenfalls am Stickstoffatom durch eine oder zwei Alkylgruppen substituiert), Acylamino- (gegebenenfalls am Stickstoffatom durch eine Alkylgruppe substituiert), Alkoxycarbonyl-, Carboxy-, Sulfo- oder Cyanogruppe bedeuten, deren Enantiomere, Diastereoisomere sowie deren Additionssalze mit einer oder mehreren pharmazeutisch annehmbaren Säuren oder einer oder mehreren pharmazeutisch annehmbaren Basen.

12. Verbindungen der Formel (I) nach einem der Ansprüche 1 bis 11, worin Alk eine Alkylenkette bedeutet, deren Enantiomere, Diastereoisomere sowie deren Additionssalze mit einer oder mehreren pharmazeutisch annehmbaren Säuren oder einer oder mehreren pharmazeutisch annehmbaren Basen.

13. Verbindungen der Formel (I) nach einem der Ansprüche 1 bis 12, worin W in der Position 4 der Phenylgruppe steht, deren Enantiomere, Diastereoisomere sowie deren Additionssalze mit einer oder mehreren pharmazeutisch annehmbaren Säuren oder einer oder mehreren pharmazeutisch annehmbaren Basen.

14. Verbindungen der Formel (I) nach einem der Ansprüche 1 bis 13, worin W eine Cyanogruppe bedeutet, deren Enantiomere, Diastereoisomere sowie deren Additionssalze mit einer oder mehreren pharmazeutisch annehmbaren Säuren oder einer oder mehreren pharmazeutisch annehmbaren Basen.

15. Verbindungen der Formel (I) nach einem der Ansprüche 1 bis 13, worin W eine Gruppe -N(R₁)-Z₁-R₂ bedeutet, deren Enantiomere, Diastereoisomere sowie deren Additionssalze mit einer oder mehreren pharmazeutisch annehmbaren Säuren oder einer oder mehreren pharmazeutisch annehmbaren Basen.

16. Verbindungen der Formel (I) nach einem der Ansprüche 1 bis 13, worin W eine Gruppe -Z₂-NR₁R₂ bedeutet, deren Enantiomere, Diastereoisomere sowie de ren Additionssalze mit einer oder mehreren pharmazeutisch annehmbaren Säuren oder einer oder mehreren pharmazeutisch annehmbaren Basen.

17. Verbindungen der Formel (I) nach einem der Ansprüche 1 bis 13 oder 16, worin Z₂ eine Gruppe bedeutet ausgewählt aus -C(O)-, -C(S)-, -C(NR₄)- und -S(O)ᵣ, deren Enantiomere, Diastereoisomere sowie deren Additionssalze mit einer oder mehreren pharmazeutisch annehmbaren Säuren oder einer oder mehreren pharmazeutisch annehmbaren Basen.

18. Verbindungen der Formel (I) nach einem der Ansprüche 1 bis 13 oder 16, worin Z₂ eine Bindung darstellt, deren Enantiomere, Diastereoisomere sowie deren Additionssalze mit einer oder mehreren pharmazeutisch annehmbaren Säuren oder einer oder mehreren pharmazeutisch annehmbaren Basen.

19. Verbindungen der Formel (I) nach einem der Ansprüche 1 bis 13 oder 15, worin Z₁ eine Gruppe bedeutet ausgewählt aus -C(O)-, -C(S)-, *-C(O)-N(R₃)-, *-C(S)-N(R₃)-, *-C(O)-O und -S(O)₂-, deren Enantiomere, Diastereoisomere sowie deren Additionssalze mit einer oder mehreren pharmazeutisch annehmbaren Säuren oder einer oder mehreren pharmazeutisch annehmbaren Basen.

20. Verbindungen der Formel (I) nach einem der Ansprüche 1 bis 13 oder 15 bis 19, worin R₁, R₂, R₃ und R₄, die gleich oder verschieden sind, ein Wasserstoffatom oder eine Gruppe ausgewählt aus Cycloalkyl; Alkoxy; gegebenenfalls substituiertem Phenyl; Naphthyl; einer Heteroarylgruppe; und einer Alkylgruppe, die gegebenenfalls substituiert ist
- entweder durch eine gegebenenfalls substituierte Phenylgruppe,
- oder durch eine Cycloalkylgruppe,
- oder durch eine Heterocycloalkylgruppe,
- oder durch eine Heteroarylgruppe,
- oder durch eine oder zwei Alkoxygruppe
- oder durch eine Phenyloxygruppe;
deren Enantiomere, Diastereoisomere sowie deren Additionssalze mit einer oder mehreren pharmazeutisch annehmbaren Säuren oder einer oder mehreren pharmazeutisch annehmbaren Basen.

21. Verbindungen der Formel (I) nach einem der Ansprüche 1 bis 13, 15 bis 17 oder 19, worin W eine Gruppe bedeutet ausgewählt aus -N(R₁)-C(O)-NR₂R₃; -N(R₁)-C(S)-NR₂R₃; -C(O)-NR₁R₂ und -C(S)-NR₁R₂; worin R₁ und R₂ oder R₂ und R₃ gemeinsam mit dem oder den sie tragenden Stickstoffatomen eine Heterocycloalkylgruppe oder eine Piperidinopiperidinylgruppe bilden, deren Enantiomere, Diastereoisomere sowie deren Additionssalze mit einer oder mehreren pharmazeutisch annehmbaren Säuren oder einer oder mehreren pharmazeutisch annehmbaren Basen.

22. Verbindungen der Formel (I) nach einem der Ansprüche 1 bis 13 oder 16 bis 18, worin W eine Gruppe -Z₂-NR₁R₂ bedeutet, worin Z₂ eine Bindung darstellt; R₁ und R₂ zusammen mit dem sie tragenden Stickstoffatom eine Heteroarylgruppe bilden oder R₁ ein Wasserstoffatom oder eine Alkylgruppe und R₂ eine Aryl- oder Heteroarylgruppe bedeuten, deren Enantiomere, Diastereoisomere sowie deren Additionssalze mit einer oder mehreren pharmazeutisch annehmbaren Säuren oder einer oder mehreren pharmazeutisch annehmbaren Basen.

23. Verbindungen der Formel (I) nach einem der Ansprüche 1 bis 13, 16, 17 oder 21, worin W eine Gruppe -C(O)-NR₁R₂ bedeutet, worin R₁ und R₂ gemeinsam mit dem sie tragenden Stickstoffatom eine Gruppe bilden ausgewählt aus Piperazinyl, das gegebenenfalls durch eine Alkyl- oder Benzylgruppe substituiert ist; Piperidinyl, das gegebenenfalls durch eine Alkyl- oder Benzylgruppe substituiert ist; Morpholinyl; Azepanyl; Thiomorpholinyl; Octahydrocyclopentapyrrolyl; Dihydrochinolinyl; und Tetrahydrochinolinyl, deren Enantiomere, Diastereoisomere sowie deren Additionssalze mit einer oder mehreren pharmazeutisch annehmbaren Säuren oder einer oder mehreren pharmazeutisch annehmbaren Basen.

24. Verbindungen der Formel (I) nach einem der Ansprüche 1 bis 13, 16, 17 oder 20, worin W eine Gruppe -C(O)-NR₁R₂ bedeutet, worin R₁ und R₂ unabhängig voneinander eine Alkylgruppe oder ein Wasserstoffatom bedeuten, deren Enantiomere, Diastereoisomere sowie deren Additionssalze mit einer oder mehreren pharmazeutisch annehmbaren Säuren oder einer oder mehreren pharmazeutisch annehmbaren Basen.

25. Verbindungen der Formel (I) nach einem der Ansprüche 1 bis 13. 15, 19 oder 20, worin W eine Gruppe -N(R₁)-C(O)-R₂ bedeutet, worin R₁ und R₂ unabhängig voneinander eine Alkylgruppe oder ein Wasserstoffatom bedeuten, deren Enantiomere, Diastereoisomere sowie deren Additionssalze mit einer oder mehreren pharmazeutisch annehmbaren Säuren oder einer oder mehreren pharmazeutisch annehmbaren Basen.

26. Verbindung der Formel (I) nach einem der Ansprüche 1 bis 14, nämlich 4-(3-Hexahydrocyclopenta[-c]pyrrol-2(1H)-ylpropoxy)-benzonitril-, dessen Enantiomere, Diastereoisomere sowie dessen Additionssalze mit einer pharmazeutisch annehmbaren Säure.

27. Verbindung der Formel (I) nach einem der Ansprüche 1 bis 13, 16, 17, 20 oder 24, nämlich 4-(3-Hexahydrocyclopenta[c]pyrrol-2(1H)-ylpropoxy)-benzamid, dessen Enantiomere, Diastereoisomere sowie dessen Additionssalze mit einer pharmazeutisch annehmbaren Säure.

28. Verbindung der Formel (I) nach einem der Ansprüche 1 bis 13, 16, 17, 20 oder 24, nämlich 4-[3-(Hexahydrocyclopenta[*c*]pyrrol-2(1H)-yl)propoxy]-*N*-methyl-benzamid, dessen Enantiomere, Diastereoisomere sowie dessen Additionssalze mit einer pharmazeutisch annehmbaren Säure.

29. Verbindung der Formel (I) nach einem der Ansprüche 1 bis 13, 16, 17, 20 oder 24, nämlich 4-[3-(Hexahydrocyclopenta[c]pyrrol-2(1H)-yl)propoxy]-N,N-dimethyl-benzamid, dessen Enantiomere, Diastereoisomere sowie dessen Additionssalze mit einer pharmazeutisch annehmbaren Säure.

30. Verbindung der Formel (I) nach einem der Ansprüche 1 bis 13, 15, 19, 20 oder 25, nämlich *N*-[4-(3-Hexahydrocyclopenta[*c*]pyrrol-2(1*H*)-ylpropoxy)-phenyl]-acetamid, dessen Enantiomere, Diastereoisomere sowie dessen Additionssalze mit einer pharmazeutisch annehmbaren Säure.

31. Verfahren zur Herstellung der Verbindungen der Formel (I) nach Anspruch 1, **dadurch gekennzeichnet, daß** man als Ausgangsprodukt eine Verbindung der Formel (II) verwendet: 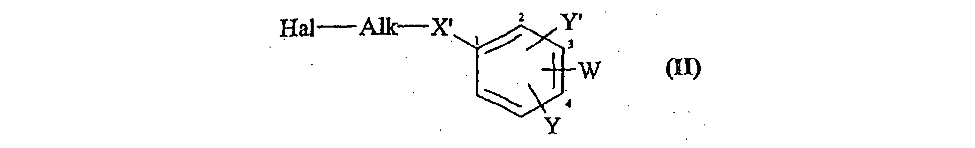 in der:
Alk die bezüglich der Formel (I) angegebenen Bedeutungen besitzt, Hal ein Halogenatom darstellt, X ein Sauerstoffatom, Schwefelatom oder eine Gruppe -N(p)- bedeutet, worin (p) ein Wasserstoffatom, eine klassische Schutzgruppe für das Stickstoffatom oder eine Alkylgruppe darstellt und W, Y und Y' die bezüglich der Formel (I) angegebenen Bedeutungen besitzen,
welche Verbindung der Formel (II) nach der eventuell durchgeführten Abspaltung der Schutzgruppe in basischem Medium mit einem Bicyclus der Formel (III) kondensiert wird: 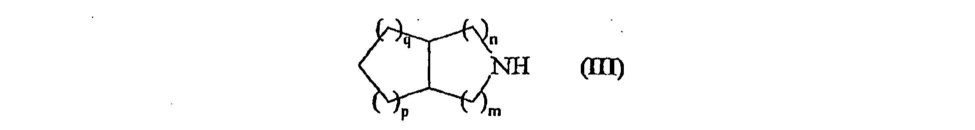 in der:
n, m, p und q die bezüglich der Formel (I) angegebenen Bedeutungen besitzen zur Bildung der Verbindung der Formel (I),
■ welche Verbindung der Formel (I) dann, wenn W eine Cyanogruppe darstellt, gegebenenfalls mit Natriumhydroxid oder Kaliumhydroxid umgesetzt wird, zur Bildung der Verbindung der Formel (I/b): 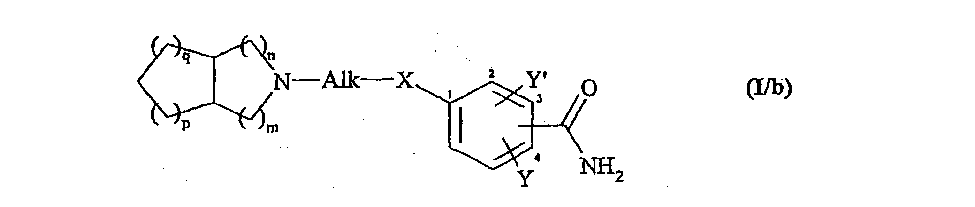 einem Sonderfall der Verbindungen der Formel (I), worin Alk, n, m, p, q, X, Y und Y' die bezüglich der Formel (I) angegebenen Bedeutungen besitzen,
welche Verbindungen der Formel (I)
- gegebenenfalls mit Hilfe einer klassischen Reinigungsmethode gereinigt werden können,
- welche man gegebenenfalls mit Hilfe einer klassischen Trennmethode in ihre Stereoisomeren auftrennt,
- welche man gewünschtenfalls in ihre Additionssalze mit einer oder mehreren pharmazeutisch annehmbaren Säuren oder einer oder mehreren pharmazeutisch annehmbaren Basen überführt,
mit der Maßgabe, daß:
- man in irgendeinem geeigneten Augenblick im Verlauf des oben beschriebenen Verfahrens die Carbonyl-, Thiocarbonyl-, Amino- oder Alkylaminogruppe(n) des Ausgangsmaterials (II) schützen kann und nach der Kondensation zum Zwecke der Synthese von den Schutzgruppen befreien kann und
- man die Reaktionsteilnehmer (II) und (III) mit Hilfe an sich bekannter, in der Literatur beschriebener Verfahrensweisen herstellt.

32. Pharmazeutische Zubereitung enthalten als Wirkstoff mindestens eine Verbindung nach einem der Ansprüche 1 bis 30 allein oder in Kombination mit einem oder mehreren inerten, nichttoxischen, pharmazeutisch annehmbaren Trägermaterialien oder Hilfsstoffen.

33. Pharmazeutische Zubereitung nach Anspruch 32, enthaltend mindestes einen Wirkstoff nach einem der Ansprüche 1 bis 30 nützlich als Arzneimittel zur Behandlung von Erkenntnisdefiziten, die mit dem Altern des Gehirns und neurodegenerativen Erkrankungen verknüpft sind, sowie zur Behandlung von Gemütsstörungen, konvulsiven Krisen, des Hyperaktivitätssyndroms mit Aufmerksamkeitsdefiziten, Fettsucht und Schmerzen.

34. Pharmazeutische Zubereitung nach Anspruch 32, enthaltend mindestens einen Wirkstoff nach einem der Ansprüche 1 bis 30, nützlich als Arzneimittel zur Behandlung von kognitiven Defiziten, die mit der Alzheimerschen Krankheit, der Parkinsonschen Krankheit, der Pickschen Krankheit, der Korsakoff-Krankheit, frontalen und subkortikalen Demenzen vaskulären oder anderen Ursprungs geeignet sind.

35. Verwendung einer pharmazeutischen Zubereitung nach Anspruch 32, die mindestens einen Wirkstoff nach einem der Ansprüche 1 bis 30 enthält, für die Herstellung von Arzneimitteln, die nützlich sind bei der Behandlung von kognitiven Defiziten, die mit dem Altern des Gehirns und neurodegenerativen Erkrankungen verknüpft sind, sowie zur Behandlung von Gemütsstörungen, konvulsiven Krisen, dem Hyperaktivitätssyndrom mit Aufmerksamkeitsdefiziten, der Fettsucht und von Schmerzen.

36. Verwendung der pharmazeutischen Zubereitung nach Anspruch 32, die mindestens einen Wirkstoff nach einem der Ansprüche 1 bis 30 enthält, für die Herstellung von Arzneimitteln, die nützlich sind bei der Behandlung von kognitiven Defiziten, die mit der Alzheimerschen Krankheit, der Parkinsonschen Krankheit, der Pickschen Krankheit der Korsakoff-Krankheit und frontalen und subkortikalen Demenzen vaskulären oder andersartigen Ursprungs verknüpft sind.
